# EUROPEAN PATENT SPECIFICATION

(11) **EP 2 259 773 B1**
(45) Date of publication and mention of the grant of the patent: **17.07.2013**
(21) Application number: 09718249.7
(22) Date of filing: 05.03.2009
(51) Int. Cl.: A61K 9/00, A61K 31/7048

(54) **MACROLIDE COMPOSITIONS HAVING IMPROVED TASTE AND STABILITY**
MAKROLIDZUSAMMENSETZUNGEN MIT VERBESSERTEM GESCHMACK UND ERHÖHTER STABILITÄT
COMPOSITIONS DE MACROLIDE AYANT UN GOÛT ET UNE STABILITÉ AMÉLIORÉS

(30) Priority: 05.03.2008 EP 08004085
(43) Date of publication of application: 15.12.2010
(73) Proprietor: PARI Pharma GmbH, 82319 Starnberg (DE)
(72) Inventor: KELLER, Manfred, 81379 München (DE); CORBANIE, Evy, 80807 München (DE)
(74) Representative: Beckmann, Claus
(86) International application number: PCT/EP2009/001572
(87) International publication number: WO 2009/109390

(56) References cited:
- WO-A-02/03998
- WO-A-2005/002592
- WO-A-2008/025560
- US-A- 5 633 006

## Description

### FIELD OF THE INVENTION

The invention relates to liquid aqueous pharmaceutical compositions for administration as an aerosol, comprising a macrolide antibiotic component, which are useful for pulmonary, nasal, or topical application. The compositions are especially useful for the prevention or treatment of diseases affecting the airways, such as the lungs, bronchi, and sinunasal cavities, or treatment of infections of the oropharyngeal region or the nose. The invention also relates to solid pharmaceutical compositions for preparing aqueous solutions for nebulisation as respirable aerosols.

### BACKGROUND OF THE INVENTION

Many diseases are caused by inflammations of bacterial origin which can be treated with antibiotics. Macrolides belong to a class of antibiotics with a widespread use for local, topical and systemic application. Chemically, they are cyclic molecules consisting of a lactone ring and glycosidic bonds to sugars or aminosugars. The macrolides differ from each other due to a different size of the lactone ring, which can consist of 14, 15 or 16 C-atoms, and/or due to a different nature of the sugar, which is in most cases cladinose or desosamine. Most macrolides have a very bad and bitter taste, a poor aqueous stability, and a poor oral bioavailability (about 10 - 40%) which, additionally, is highly variable. In many cases undesired gastrointestinal side effects occur after oral administration. On the other hand, the newer macrolides, such as azithromycin, offer some interesting therapeutic features such as a broader antibiotic spectrum combined with an anti-inflammatory and immunomodulatory effect, good local tolerability and tissue penetration. Thus, topical administration would offer advantages, on the condition that the bad taste and poor stability of aqueous systems could be overcome.

The delivery of therapeutic compounds to the skin, ears or eyes is a common and simple option to target drugs to the site where drugs are needed and to overcome undesired systemic side effects. It would also be desirable to deliver drugs to the upper and lower respiratory tract, but this requires sophisticated drug delivery systems.

In general, drug substances can be delivered to the respiratory system as aerosolised dry powders or liquids, the liquids representing either solutions or dispersions, such as drug suspensions. Various devices have been developed to convert a liquid or solid composition into an aerosol and to enable inhalation. One of the most important requirements for any such device is that it is capable of achieving a particle size of the aerosol which will allow deposition at the target site, i.e. the designated site of action or of absorption. Depending on whether the drug should be delivered to the nose, paranasal cavities, the oropharyngeal area, bronchi or to the deep lungs, the optimal droplet or particle size for typical formulations may vary from about 10 µm down to below 1 µm; larger particles may be useful if their density is very low.

Metered-dose inhalers (MDIs) deliver a measured dose of the drug in the form of a solution or suspension of small liquid or solid particles, which is dispensed from the inhaler by a propellant under pressure. Such inhalers are placed into the nose or mouth and activated to release drug. For a reliable pulmonary deposition, this requires a certain amount of coordination and is known to be highly variable. Spacers, or spacing devices, which are available for use with some aerosol inhalers, extend the space between the inhaler and the mouth. This reduces the speed at which the aerosol travels to the back of the mouth, allowing more time for the propellant to evaporate and therefore reducing the impact of the propellant on the back of the mouth, which can cause irritation, and enabling a higher proportion of the particles of the drug to be inhaled. There is also less need to coordinate the inhalation manoeuvre with activation of the inhaler. Breath-activated inhalers deliver the drug, in the form of an aerosol or a dry powder, only when the user places his mouth over the outlet and breathes in. This obviates the need to coordinate the inhalation manoeuvre with depressing the dispenser. The dose of drug will still be measured or metered, and is not dependent on the size of breath taken. However, metered dose inhalers in combination with spacers can deliver only small drug quantities in the range of about 0.02 - 1 mg/puff.

Dry powder inhalers (DPIs), on the other hand, are loaded with portions of the drug substance in form of a powder formulation. The unit doses may be accommodated in small capsules as for example in the commercially available devices known as "Spin-haler" and "Rotahaler". Upon activation of these inhalers, the capsule is punctured. By subsequently taking a breath, a turbulent air flow is generated which disperses the powder in the air flow so that it can be inhaled. Another device known as "Discus" or "Accuhaler" is fitted with a blister foil that contains measured doses of the powder formulation; other devices may use a bulk reservoir and integrated metering system such as the "Turbuhaler". Typically, DPIs are bolus delivery systems meaning that a defined dose is metered and delivered upon a deep breath. The drug is mostly dispersed in a carrier such as lactose and the dose delivered per single actuation is typically in a range of about 0.01 - 1 mg, whereas the total powder weight per actuation may vary from about 5 - 20 mg.

In most cases, the droplet or particle distribution pattern of MDIs, DPIs and many nebuliser systems is broad and consists of very small up to very large particles characterized by a broad geometric standard deviation (GSD) larger than about 2. Large particles or droplets can cause undesired oropharyngeal deposition and particles or droplets smaller than about 3 µm have a high probability to be either systemically absorbed or exhaled.

Aqueous, i.e. water-based, solutions and suspensions are usually inhaled with nebulisers. Various types of nebulisers are commercially available or presently being developed. A traditional type is the jet nebuliser, which is still being used extensively. More recently, ultrasonic and vibrating membrane-type nebulisers were developed. Contrary to MDIs and DPIs, nebulisers are non-bolus systems, since the drug is administered during regular breathing cycles which can last up to 30 min depending on the volume and type of nebuliser used. Hence, these systems are capable to deliver drugs in very low and high doses upon spontaneous breathing and offer therefore some advantages over MDIs and DPIs particularly when drugs in doses > 1 mg must be delivered into the respiratory tract.

While traditional inhalation therapies were primarily directed to the prevention and treatment of allergic and inflammatory diseases and conditions of the respiratory system including asthma and obstructive bronchitis, novel therapeutical approaches have been developed more recently. For instance, the local treatment of pulmonary infections with antibiotics has been suggested and, with tobramycin being the first antibiotic approved for this use, successfully introduced to the therapy of certain severe or even life-threatening types of infection. Tobramycin is supplied as Tobi^{™}, a sterile, clear, slightly yellow, non-pyrogenic, aqueous solution with the pH and salinity adjusted specifically for administration by a compressed air driven reusable nebuliser. It is approved in a dose of 300 mg/5 ml for the treatment of cystic fibrosis patients infected with *Pseudomonas aeruginosa* using the PARI LC PLUS^{™} nebuliser.

Other pulmonary antibiotic therapies have been proposed in the scientific and patent literature. For instance, WO 02/03998 discloses inhalable formulations of macrolide antibiotics, such as erythromycylamine, for delivery by aerosolisation. The concentrated erythromycylamine formulations contain an amount of erythromycylamine effective to treat infections caused by susceptible bacteria. Unit dose devices having a container comprising a formulation of the macrolide antibiotic in a physiologically acceptable carrier are also described. The document further discloses methods for treatment of pulmonary infections by such formulations delivered as an aerosol having mass median aerodynamic diameter predominantly between 1 and 5 micrometers.

In WO 00/35461, a method for the treatment of severe chronic bronchitis (bronchiectasis) using a concentrated aminoglycoside antibiotic formulation is disclosed. The method includes delivering the antibiotic to the lungs' endobronchial space including alveoli in an aerosol or dry powder having a mass medium diameter predominately between 1 and 5 µm. The method comprises the administration of the antibiotic at a concentration one to ten thousand times higher than the minimal inhibitory concentration of the target organism. Preferably, the method comprises the endobronchial administration of aerosolized tobramycin to treat pseudomonal infections in severe chronic bronchitis patients.

A wide variety of gram-negative bacteria cause severe pulmonary infections, and many of these bacteria are or become resistant to commonly used or specialty antibiotics including tobramycin, and require treatment with new types of antibiotics. The pulmonary infections caused by gram-negative bacteria are particularly dangerous to patients who have decreased immunoprotective responses, such as cystic fibrosis (CF) and HIV patients, patients with chronic obstructive pulmonary disease (COPD), bronchiectasis or those on mechanical ventilation. Thus, bacterial respiratory infections caused by resistant bacteria remain a major problem, particularly in CF, COPD and HIV patients or those receiving immunosuppressive drugs. For example, chronic pulmonary infection with *Pseudomonas aeruginosa* in patients with cystic fibrosis is a major cause of their high mortality.

In order to address the continuous need for an effective therapy for treatment of acute and chronic pulmonary bacterial infections caused by gram-negative bacteria and particularly those caused, for example, by *Burkholderia cepacia, Stenotrophomonas maltophilia, Alcaligenes xylosoxidans,* and multidrug resistant *Pseudomonas aeruginosa,* WO 02/051356 proposes the local therapy of the respiratory system by delivering a concentrated formulation of the monobactam antibiotic aztreonam as an inhalable aerosol, or as a dry powder formulation. According to the document, about I to 250 mg of aztreonam may be dissolved in 1 to 5 ml of saline or another aqueous solution. The formulation is delivered to the lung endobronchial space as an aerosol having mass median average diameter particles predominantly between 1 and 5 micrometers, using a nebuliser capable of atomizing the aztreonam solution into droplets or particles of the required sizes. Alternatively, for the delivery of a dry inhalable powder, aztreonam is milled or spray dried to particle sizes of 1 to 5 micrometers.

Another anti-infective agent suggested for inhalation therapy is azithromycin. Azithromycin is a macrolide antibiotic with activity against common respiratory pathogens such as *Streptococcus pneumoniae* and *Haemophilus influenzae.* It has potential anti-inflammatory effects in the management of chronic *Pseudomonas aeruginosa* respiratory tract infection in CF patients. There is some evidence that short-term use in both adults and children with CF results in improved clinical and quality of life parameters. The impact of longer-term use is unknown. Azithromycin may act synergistically with other agents against a range of CF pathogens, enhancing their *in vitro* activity. It is not known if this will result in improved clinical efficacy. In general, it has been well tolerated by CF patients when given orally.

It has been suggested, e.g. by A. J. Hickey et al. (J. Aerosol Med. 19 (1), 2006, 54-60), that azithromycin should be used in the local treatment of pulmonary infections. Hickey *et al.* further describe experiments in which aqueous solutions of this anti-infective agent in various concentrations have been more or less efficiently nebulised using three conventional jet nebulisers. The tests were performed with a commercially available freeze-dried azithromycin powder formulation to be dissolved prior to (parenteral) administration. Zithromax^{®} is a lyophilised azithromycin preparation in a 10 ml vial containing citric acid and sodium hydroxide as excipients. Reconstitution according to label directions results in a solution of about 500 mg / 5 ml equivalent to about 100 mg azithromycin / 1 ml. However, the reconstituted solution is only stable for 24 hours at or below room temperature (30°C) or for 7 days if stored under refrigeration (5°C) (Zitromax^{®} IV U.S. Physician Prescribing Information; August 2007 revision).

In clinical practice, however, it is not only the aerosolisation which is needed for therapeutic success. In addition, the administration of the drug product must be acceptable to patients in order to achieve compliance. In the case of azithromycin, the acceptability of a simple - perhaps buffered - aqueous solution for inhalation is rather doubtful as the poor and bitter taste of the drug substance severely compromises the usefulness of such a formulation. Also other macrolides, such as clarithromycin, are difficult to formulate appropriately for inhalation because of poor taste. Additionally, solutions for inhalation should contain high concentrations of the drugs, as it is known that inhalation times exceeding about 5 - 10 min decrease patient acceptance.

In general, formulating aqueous compositions which are useful for nebulisation can be challenging, depending on the physical, chemical, and organoleptic properties of the active agent. Clearly, aqueous solutions are usually most preferred for nebulisation, but not often easily achievable. Two aspects, namely poor aqueous solubility and poor aqueous stability, often represent problems to formulate solutions for nebulisation. The poor aqueous solubility of macrolides such as azithromycin has been solved by adding cosolvents such as propylene glycol as described in US 2003/0092640. Another approach has been illustrated in US 2006/0252711, where a formulation for treatment of ocular infections has been described. In the latter case, azithromycin was dissolved in an oily vehicle of linear medium-chain fatty acid triglycerides. However, these approaches cannot easily be transferred to formulations for inhalation, due to possible toxicity of such excipients in the lungs and due to problems to obtain small droplet sizes upon nebulisation due to increased viscosity.

Poor aqueous stability has generally been circumvented by presenting the formulation in a dry powder form that is only reconstituted at the time of use. For example, azithromycin for intravenous application has been formulated and commercialized as a freeze-dried powder, which should be used within 24 h after reconstitution. In another approach, azithromycin is presented as a suspension, as for example described in US 6,861,413 (oral suspensions) and in US 6,239,113 and US 6,569,443 (suspensions for ocular application). The latter approach is less suited for formulations for nebulisation, as it is known that it is more difficult to entrain particles larger than 1 µm in fine aerosol droplets, resulting in reduced nebulisation efficiency (Keller et al., "Nebulizer nanosuspensions: Important device and formulation interactions", Resp. Drug Delivery VIII, 2002, p. 197-206; and Luangkhot et al., "Characterisation of salbutamol solution compared to budesonide suspensions consisting of submicron and micrometer particles in the PARI LC STAR and a new PARI Electronic Nebuliser (eFlow™)", Drug Delivery to the Lungs XI, 2000, p. 14-17).

Further efforts have been made to obtain stable aqueous solutions of macrolides. In WO 02/03998, erythromycylamine has been formulated in solution for inhalation by salt formation. In this case, formulations of erythromycylamine hydrochloride, erythromycylamine sulphate, and erythromycylamine acetate at pH 7 have been shown to be stable for 16 days at 60°C. In a further approach, described in EP 1 075 837, azithromycin has been stabilized in aqueous solutions for application in the eye by adding appropriate amounts of citric acid/phosphate buffer ratio. Also US 7,056,893 describes stabilised solutions of azithromycin in combination with a citric acid buffer for treatment of ocular infections. However, the improvement of taste of the drug has not been aimed for in these approaches.

Interestingly, several drug substances which have recently been suggested as being potentially useful for inhalation therapy have a rather poor taste. Moreover, it has been found by the inventors that such poor taste may be as unpleasant when inhaling a nebulised solution of the respective compound as in the case of oral administration. The unpleasant taste results in the reduction of patient compliance, which influences the therapy. Therefore, taste-masking is one of the crucial parameters in the development of pharmaceutical compositions for aerosol therapy.

As poor organoleptic properties of active agents are an already well-known problem in oral drug delivery, most prior art relating to the improvement or masking of the poor taste of drug substances relates to standard oral dosage forms such as tablets and capsules. A simple and usually rather effective method of formulating such compounds is to coat the drug particles or the whole dosage form with a saliva-resistant coating. However, the use of coatings for taste-masking is not feasible for compositions for aerosolisation.

In another approach to mask the taste of bitter active compounds, the use of divalent cations has been described. For example, WO 03/032973 describes the taste masking of paracetamol with magnesium salts, magnesium oxide and magnesium hydroxide. In EP 0 582 396 alkaline earth oxides and hydroxides and in US 2007/0185194 magnesium, sodium and calcium salts have been described for taste-masking of azithromycin in dry powder form. Taste-masking of levofloxacin in a liquid formulation for inhalation through complexation with divalent cations has been described in WO 06/125132. However, none of these applications described an improved stability of azithromycin when formulated and stored as an aqueous solution.
US5633006 describes a taste masking mix for masking bitterness of macrolides and uses alkaline earth oxides for this purpose. The authors suggests using additional optional components, such as aldonic acids, preferably calcium gluconate.
WO2005002592 addresses the problem of taste-masking and suggests to use NaCl, as well as calcium gluconate as optional taste masking agent

Thus, there is a need for taste-masked aqueous pharmaceutical compositions containing macrolide antibiotics and showing sufficient storage stability when dissolved in water or in a salt solution. Such compositions should be topically well-tolerable and applicable without causing undesired side effects. Furthermore, such aqueous compositions should be suitable for aerosolisation and for the prevention, management or treatment of airway diseases and conditions. Such a composition in combination with a sophisticated nebuliser should have improved acceptability for patients compared to currently available macrolide compositions that are used off-label for nebulisation. Improved acceptability leads to better therapy adherence and subsequently to more efficient treatments. One of the particular objects of the present invention is to provide aqueous compositions having both reasonable shelf-life, i.e. storage stability, and acceptable taste without local irritation potency upon topical administration in the oropharyngeal region or the nose or upon inhalation using highly efficient nebulisers. Further objects will become clear on the basis of the following description and the patent claims.

### SUMMARY OF THE INVENTION

The invention provides liquid aqueous macrolide compositions for administration as an aerosol. The compositions are stable when stored at 4 - 8°C for up to 3 years and at 25°C for several months. The composition constituents not only improve the stability of the dissolved macrolide but simultaneously improve the bad taste and topical tolerability of the formulations when administered in the oropharyngeal region or the nose or respiratory tract.

More specifically, the invention provides a liquid aqueous pharmaceutical composition for administration as an aerosol to the respiratory tract, nose or oropharyngeal region comprising (i) a macrolide having a poor taste and poor chemical stability in aqueous solution; (ii) at least one salt selected from the group consisting of sodium gluconate, sodium aspartate, sodium acetate, sodium lactate, sodium succinate, sodium maleate, magnesium gluconate, magnesium aspartate, magnesium citrate, magnesium acetate, magnesium lactate, magnesium succinate, and magnesium maleate; and (iii) a taste-masking agent different from said salt; wherein (a) the concentration of said macrolide in the composition is in the range of about 0.25 wt.% to about 15 wt.-%; (b) the molar ratio of said macrolide : said salt is in the range from about 1 : 0.5 to about 1 : 100; (c) the pH of the composition is in the range of about 3 to 9; and (d) the osmolality of the composition is in the range of about 150 mOsmol/kg to about 1500 mOsmol/kg.

The pharmaceutical composition can be used for aerosolisation via a nebuliser producing a pharmaceutical aerosol for nasal, sinunasal or pulmonary administration. This aerosol comprises a dispersed liquid phase and a continuous gas phase. The dispersed liquid phase essentially consists of aqueous droplets preferably having a mass median diameter from about 1.5 to about 6 µm. The droplets of the dispersed phase comprise the macrolide antibiotic.

Thus, the pharmaceutical composition can be used for the manufacture of a medicament for the prophylaxis or treatment of diseases or conditions of the lower and upper respiratory tract, or infections or inflammation of the nose or oropharyngeal regions.

It has been surprisingly found that compositions for administration as an aerosol wherein the poor taste of a water-soluble macrolide is masked, covered or improved with particular effectiveness can be provided by using selected sodium and magnesium salts at specific macrolide : salt ratios. Furthermore, it was surprisingly found that only these specific salts at these specific ratios provide the additional advantage of both increased chemical and physical stability and taste-masking of macrolides in aqueous systems. The salts can be used separately or in combination with each other. Also surprisingly, precipitation was observed when using calcium salts, such as calcium chloride, but can be avoided by use of the aforementioned specific salts.

The invention further provides a method of generating an aerosol, said method comprising (a) providing a liquid aqueous composition as defined above; (b) providing an aerosol generator capable of aerosolising the composition; and (c) operating said aerosol generator to aerosolise the composition.

Moreover, the invention also provides a solid pharmaceutical composition for preparing such liquid compositions. The solid composition comprises (i) a macrolide having a poor taste and poor chemical stability in aqueous solution; (ii) at least one salt selected from the group consisting of sodium gluconate, sodium aspartate, sodium acetate, sodium lactate, sodium succinate, sodium maleate, magnesium gluconate, magnesium aspartate, magnesium citrate, magnesium acetate, magnesium lactate, magnesium succinate, and magnesium maleate; and (iii) a taste-masking agent different from said salt. The solid composition is dissolvable or dispersible in an aqueous liquid solvent so that an effective dose of the macrolide antibiotic compound is dissolvable or dispersible in a volume of not more than about 10 ml, and preferably not more than about 5 ml of the solvent.

### BRIEF DESCRIPTION OF THE FIGURES

**Figure 1** shows the change of the azithromycin concentration in aqueous solutions where azithromycin was combined with the stabilising salts magnesium gluconate, magnesium aspartate and magnesium citrate in comparison with magnesium chloride, magnesium sulphate and sodium chloride, and stored at 25°C.
**Figure 2** shows the change of the azithromycin concentration in aqueous solutions where azithromycin was combined with the stabilising salts magnesium gluconate, magnesium aspartate and magnesium citrate in comparison with magnesium chloride, magnesium sulphate and sodium chloride, and stored at 40°C
**Figure 3** shows the change of the azithromycin concentration in aqueous solutions where azithromycin was combined with the stabilising salts magnesium acetate, magnesium lactate, sodium gluconate, sodium succinate, sodium maleate and sodium aspartate, and stored at 25°C
**Figure 4** shows the predicted 5°C storage stability of azithromycin dihydrate in a solution containing magnesium gluconate, based on accelerated stability evaluations at 25°C, 40°C and 70°C.

### DETAILED DESCRIPTION OF THE INVENTION

The compositions of the invention contain at least one salt selected from the group consisting of sodium gluconate, sodium aspartate, sodium acetate, sodium lactate, sodium succinate, sodium maleate, magnesium gluconate, magnesium aspartate, magnesium citrate, magnesium acetate, magnesium lactate, magnesium succinate, and magnesium maleate. It was surprisingly found that these salts simultaneously provide taste-masking and stabilisation of the macrolide.

Among the specific salts indicated above, sodium gluconate, magnesium gluconate, magnesium citrate, sodium succinate, sodium maleate, magnesium succinate, and magnesium maleate are particularly preferred.

The above salts may be used as such or in the form of "hydrogen salts", such as magnesium hydrogen citrate. The above salts may also be used in the form of hydrates, such as magnesium gluconate dihydrate, or other solvates thereof.

The compositions of the invention are used for the preparation of pharmaceutical aerosols for nasal, sinunasal or pulmonary administration comprising a dispersed liquid phase and a continuous gas phase. The compositions can also be used for topical application in the oropharyngeal region or the nose.

The dispersed liquid phase essentially consists of aqueous droplets preferably having a mass median diameter from about 1.5 to about 6 µm. The droplets of the dispersed phase comprise the macrolide having a poor taste and a poor aqueous stability, and have a pH from about 3 to 9.

The aerosols comprise a dispersed liquid phase and a continuous gas phase. Such aerosols are sometimes referred to as "liquid aerosols" or, probably more appropriately, aerosolised liquids. It should be noted that the requirement of a dispersed liquid phase does not exclude the presence of a solid phase. In particular, the dispersed liquid phase may itself represent a dispersion, such as a suspension of solid particles in a liquid.

The continuous gas phase may be selected from any gas or mixture of gases which is pharmaceutically acceptable. For example, the gas phase may simply be air or compressed air, which is most common in inhalation therapy using nebulisers as aerosol generators. Alternatively, other gases and gas mixtures, such as air enriched with oxygen, carbon dioxide or mixtures of nitrogen and oxygen may be used (Helox^{™}). Most preferred is the use of air as continuous gas phase.

In the context of the present invention, the term active compound refers to a natural, biotechnology-derived or synthetic compound or mixture of compounds useful for the diagnosis, prevention, management, or treatment of a disease, condition, or symptom of an animal, in particular a human. Other terms which may be used as synonyms of active compound include, for example, active ingredient, active agent, active pharmaceutical ingredient, therapeutic compound, drug substance, drug, and the like.

It should be noted that many active compounds are available in various forms, e.g. as salts or solvates. Some of the forms may be water soluble while others exhibit poor water solubility. In the context of the present invention, only the water solubility of the actually incorporated form of the compound is relevant.

The active compounds used in the present invention (macrolides) do not pose a problem with respect to insufficient solubility which could make the development of aqueous formulations for inhalation difficult. It is particularly important that the aqueous solubility of the drug substance is sufficiently high in relation to its single therapeutic dose. Preferred active compounds have a solubility which allows that a single dose can be dissolved in not more than about 5 ml, preferably not more than about 2 ml, of a pharmaceutically acceptable aqueous medium or buffer system.

The macrolide used in the present invention, as such, has a poor taste. As used herein, a poor taste is a taste which could have a negative impact on the acceptability and patient compliance. According to another definition, the taste is poor if an aqueous - optionally buffered - solution of a single dose of the active compound in a volume of 0.5 up to 10 ml is regarded as poor. A poor tasting compound may taste bitter, metallic, acrid, astringent, or otherwise unpleasant.

Examples of macrolides of interest for inhalation therapy which have a poor taste include azithromycin, clarithromycin, josamycin, roxithromycin, and erythroymcin as well as ketolides, such as telithromycin. As used herein, a reference to the INN name of a compound includes all potentially applicable forms of that substance, in particular the salts, solvates, isomers, conjugates, prodrugs and derivative thereof. One of the particularly preferred macrolides antibiotics is azithromycin, including its salts and solvates, such as azithromycin dihydrate or azithromycin monohydrate ethanolate.

The composition containing the active compound, in particular azithromycin or salt or solvate thereof, is preferably used for the prophylaxis or treatment of a variety of diseases and conditions of the lower and upper respiratory, such as acute or chronic sinusitis or rhinosinusitis, oropharyngeal infections, bronchitis, pneumonia, asthma, chronic obstructive pulmonary disease (COPD), bronchiectasis, pulmonary ciliary dyskinesia, respiratory infections in HIV patients, graft rejection after lung, stem or bone marrow transplantation, bronchiolitis obliterans, pneumocystis, diffuse bronchiolitis, sarcoidosis, parenchymatic and/or fibrotic diseases or disorders including cystic fibrosis, nontuberculous mycobacterial pulmonary diseases, pulmonary nocardia infections, any pulmonary infection with or without acute exacerbations, for example due to *Streptococcus pneumoniae, Haemophilus influenzae, Pneumocystis jirovecii* or *Moraxella catarrhalis;* acute bacterial exacerbations in chronic bronchitis or in chronic obstructive pulmonary disease, for example due to *Staphylococcus aureus, Streptococcus pneumoniae, Haemophilus injluenzae, Haemophilus parainfluenzae, Pneumocystis jirovecii* or *Moraxella catarrhalis;* nosocomial pneumonia, for example due to *Staphylococcus aureus, Pseudomonas aeruginosa, Serratia marcescens, Bukholderia cepacia, Escherichia coli, Klebsiella pneumoniae, Haemophilus influenzae, Streptococcus pneumoniae, Pneumocystis jirovecii, Mycobacterium avium, Mycobacterium kansasii, Mycobacterium chelonae,* or *Mycobacterium abscessus;* community acquired pneumonia (CAP), hospital acquired pneumonia (HAP), ventilator associated pneumonia (VAP), for example due to *Staphylococcus aureus, Streptococcus pneumoniae, Haemophilus influenzae, Haemophilus parainfluenzae, Klebsiella pneumoniae, Moraxella catarrhalis, Chlamydia pneumoniae, Legionella pneumophila, Pneumocystis jirovecii* or *Mycoplasma pneumoniae;* fungal infections of the respiratory tract, for example due to *Aspergillus spp., Candida spp., or Zygomycetes spp.,* or viral infections of the respiratory tract, for example due to viruses from virus families such as *Orthomyxoviridae, Paramyxoviridae, Picornaviridae, Adenoviridae, Coronaviridae* or *Enteroviridae.*

The macrolides are lipophilic in nature and have a low degree of ionisation. This allows extensive penetration into tissues and fluids and results in a large volume of distribution. Concentrations of macrolides and ketolides in respiratory tract tissues and fluids are, in most cases, higher than concurrent serum concentrations. This extensive distribution into respiratory tissues and fluids makes predictions of pharmacodynamic activity difficult as serum concentrations, frequently used as predictors, do not necessarily provide a good indication of macrolide activity. Azithromycin shows an excellent distribution into respiratory tissues: the concentration of azithromycin in lung tissue after single oral dose administration exceeds plasma concentration by about 10 to 20-fold, in bronchial mucosa by 29-fold, in alveolar macrophages by 170-fold. Furthermore, the concentration in sputum exceeds the plasma concentration by 67-fold after multiple oral doses of azithromycin. However, the concentration of azithromycin in lung tissue and epithelial lining fluid will still be less after oral or systemic administration compared to after local administration in the respiratory system.

It has been suggested that antibiotics that concentrate in polymorphonuclear neutrophils (e.g., azithromycin, ciprofloxacin, levofloxacin, moxifloxacin) may be beneficial in the treatment of infections caused by bacteria that survive phagocytosis. Intracellular concentrations are important for defence against respiratory pathogens including *Legionella pneumophila, Chlamydia pneumoniae, Mycoplasma pneumoniae* and *Ureaplasma urealyticum.* The majority of the macrolides concentrate in the lysosomes. This is thought to occur as a result of trapping caused by the lower pH (4 to 5) found in the lysosomes compared with the cytoplasm (pH 7). The dibasic macrolides (e.g. azithromycin) display the highest concentrations in the lysosomes as the presence of two basic amine groups leads to greater ionisation and a subsequent increased ion-trapping. These agents also display a much slower efflux from phagocytes.

The macrolides are able to exert their effects due to lysosome fusion with the phagosomes, which is an essential event in the phagocytic killing process. Subsequently, high concentrations of the agents are deposited in the compartment where the pathogens reside. The polymorphonuclear leukocytes (neutrophil granulocytes) are believed to act as carriers in the transport of azithromycin to the site of infection through chemotaxis. The release of this agent from the neutrophils is enhanced by exposure to pathogens. Thus, neutrophils are vital in the delivery of azithromycin to sites of infection and play a dual role in the antibiotic infection cycle: neutrophils loaded with azithromycin target the site of infection and release the antibiotic into the interstitial space. The antibiotic then enhances the natural host defence mechanism by rendering the bacteria more susceptible to killing by the neutrophils.

Especially azithromycin is characterized by a remarkably long elimination half-life of about 60 h (up to 72 h). This feature makes the drug attractive for use in adults and children, since the regimen allows a once daily dosage. Compared to antibiotics with a distinctly shorter half-life such as other macrolides, ketolides or most of antibiotics of different classes this property generally provides the possibility of considerably lower frequencies of drug application.

In serum, azithromycin and clarithromycin do not reach the minimal inhibitory concentration (MIC) for some pathogens (e.g. *Haemophilus influenzae*); however, they effectively inhibit their growth. This may be because of the high concentrations of these agents that are achieved in tissues and fluids where they exceed the MIC. This underlines the fact that because of their unique pharmacokinetics, serum concentrations are not a good predictor of macrolide activity.

The macrolides exhibit antibacterial activity which persists after exposure. The post-antibiotic effect (PAE) of an agent is used to describe this type of persistent antibacterial activity and becomes important when the concentration of drug declines below the MIC. The existence of a long post-antibiotic effect of azithromycin against gram-positive and gram-negative bacteria extends the pharmacokinetic advantages of the drug and strongly supports the application of this azalide in the therapy of respiratory infections, and in other topical infections such as infections of the oropharyngeal region and nose. Furthermore, azithromycin exhibits concentration-dependent killing. Together with the prolonged persistent effects, this results in the fact that the Cmax : MIC ratio is the best predictor of clinical efficacy. Therefore, it seems advantageous to maximize drug concentrations to which the target pathogen is exposed by delivering higher doses, which will result in higher activity and which will allow to use longer dosing intervals. The high dosing concept may also help to suppress the occurrence of resistance formation.

Macrolides generally have a low adverse effect profile and are considered to be one of the safest classes of antibacterials currently available. Adverse effects including gastro-intestinal (GI) disorders, allergic reactions, hepatotoxicity, ototoxicity and local irritation have been reported. Nausea and diarrhoea were more common in patients receiving chronic systemic azithromycin therapy. Macrolide-associated GI intolerance is the most common adverse effect and is dose-related. GI intolerance has been reported to occur in 20 to 50% of patients receiving erythromycin but occurs less frequently with the newer macrolides (e.g. azithromycin, clarithromycin, roxithromycin). The effects of macrolides on the immune response have been described as being immunomodulatory, defined as suppressing hyperinununity and inflammation without overt immunosuppression. These effects are thought to be independent of their antimicrobial action. Macrolides have been shown to decrease mucous hypersecretion by a number of mechanisms, including blocking mucin production and inhibiting water and chloride efflux. Macrolides have been shown to reduce biofilm formation by *P. aeruginosa* (which was suggested to lead to increased drug resistance) and have additional direct effects on *P*. *aeruginosa,* including inhibition of motility, cellular adherence and expression of the major stress protein Gro-EL. Macrolides can initially enhance host defence by increasing nitric oxide production and mediators such as IL-1 and IL-2, IL-6 and granulocyte-macrophage colony-stimulating factor (GM-CSF). Long-term macrolide therapy then suppresses inflammatory mediators, including IL-8, eotaxin, tumour necrosis factor (TNF)-a and GM-CSF. They suppress T helper-2 cell (Th2) cytokines but not Thl cell cytokines, and decrease nuclear factor (NF)κβ. Macrolides reduce inflammatory cell infiltrate by decreasing adhesion molecule expression and enhancing apoptosis. A growing interest exists in exploiting their anti-inflammatory and immunomodulatory properties for certain chronic inflammatory respiratory diseases such as diffuse panbronchiolitis (DPB), asthma, cystic fibrosis (CF), chronic bronchitis, and chronic rhinosinusitis. An extensive body of *in vitro* and *ex vivo* evidence dating back over 40 years supports the anti-inflammatory properties of the macrolides.

Furthermore, azithromycin is believed to be one of the most potent agents currently available for the treatment of nontuberculous mycobacterial disease. When administered as prophylaxis once weekly to patients with advanced human immunodeficiency virus (HIV) disease, it significantly reduced the incidence of disseminated Mycobacterium avium complex (MAC) infection in these patients, who are at very high risk to develop this infection. Azithromycin, both administered once or thrice weekly, has been useful as the cornerstone of therapy for pulmonary MAC infection. It has significant *in vivo* activity against many other nontuberculous mycobacteria as well.

Azithromycin may also be of use in *Pneumocystis jirovecii* (formerly *Pneumocystis carinii*) pneumonia (PCP) prophylaxis in patients with advanced HIV disease.

The antimicrobial effect of azithromycin and clarithromycin in the treatment of upper and lower respiratory tract infections can be enhanced in an additive or synergistic way by the addition of another anti-infective from the group of aminoglycosides, such as tobramycin or amikacin, fluoroquinolones, such as levofloxacin, ciprofloxacin or gemifloxacin, peptide antibiotics, such as colistin, monobactams, such as aztreonam, pencms, such as meropenem, or antifungals, such as voriconazole, itraconazole, ketoconazole or posaconazole. For example, it has surprisingly been found by the inventors that the susceptibility of *Burkholderia cepacia* was relevantly increased when combining azithromycin with tobramycin instead of applying these antibiotics separately.

The immunomodulatory effect of azithromycin in the treatment of bronchiolitis obliterans and organ rejection after lung, bone marrow or stem cell transplantation can be enhanced in an additive or synergistic way by the preparation of a combination product with either cyclosporin A, tacrolimus, sirolimus, everolimus, mycophenolat mofetil, or rapamycin.

The anti-inflammatory effect of azithromycin in the treatment of upper and lower respiratory tract diseases can be enhanced in an additive or synergistic way by the addition of other anti-inflammatory drugs from the group of steroids, such as budesonide, fluticasone, mometasone, ciclesonide or dehydroepiandrosterone-derivates (e.g. dehydroepiandrosterone sulphate (DHEAS)), non-steroidal anti-inflammatory drugs (NSAIDs), such as ibuprofen, diclophenac or indomethacin, cromones, such as cromo-glycate or nedocromil, phosphodiesterase inhibitors, such as theophylline or roflumilast, or antioxidants, such as polyphenols.

In each case of the aforementioned combination products, the active drug compound will be selected as a pharmaceutically acceptable salt, solvate, isomer, conjugate, prodrug or derivative thereof.

The concentration of the macrolide in the liquid composition of the invention and in the dispersed phase of the aerosol prepared therefrom is in the range from about 0.25 to about 15 wt.-%, preferably from about 1 or 2 to about 10 wt.-%, such as about 1, 1.5, 2, 2.5, 3, 3.5, 4, 4.5, 5, 5.5, 6, 6.5, 7, 7.5, 8, 8.5, 9, 9.5 or 10 wt.%. When the liquid composition is used for topical administration in the oropharyngeal region or in the nose the concentrations of the macrolide is preferably in the range from about 0.2 wt.% to about 5 wt.-%.

The macrolide is poorly stable in an aqueous solution at 25 °C. As used herein, poorly stable in an aqueous solution means that the content of the drug compound decreases over a duration of 1 year by at least about 5 %, or even by at least about 10 %, when dissolved in an aqueous medium at 25 °C and at the same pH as the composition, but in the absence of any of the specific sodium or magnesium salts and taste-masking agents.

The liquid aqueous pharmaceutical composition of the present invention comprises at least one taste-masking agent other than the above specific sodium or magnesium salts. As used herein, a taste-masking agent is any pharmaceutically acceptable compound or mixture of compounds which is capable of improving the taste of an aqueous solution of a poor tasting active ingredient, regardless of the mechanism by which the improvement is brought about. For example, the taste-masking agent may cover the poor taste of the active compound, i.e. reduce the intensity in which it is perceived; or it may correct the taste by adding another - typically more pleasant - flavour to the composition so that the total organoleptic impression is improved.

The additional taste-masking excipient is preferably selected from the group of pharmaceutically acceptable sweeteners. Among the preferred sweeteners according to the invention are saccharin, aspartame, cyclamate, sucralose, acesulfame, neotame, thaumatin, and neohesperidine, including the salts and solvates thereof, e.g. the dihydrochalcone of neohesperidine, the sodium salt of saccharin, and the potassium salt of acesulfame. Again, the respective salts and solvates of the compounds mentioned herein are always included, whether specifically mentioned or not.

Particularly preferred sweeteners are aspartame at a concentration from about 0.1 to about 3 wt.-%, in particular from about 0.5 to about 2 wt.%, and saccharin sodium at a concentration from about 0.1 to about 2 wt.%, in particular from about 0.2 to about 1 wt.%. Alternatively, sugars such as, sucrose, trehalose, fructose, lactose or sugar alcohols, such as xylitol, mannitol, isomalt can be used in concentrations up to about 5 wt.-%.

Further useful taste-masking agents include pharmaceutically acceptable surfactants, alkali or alkaline earth metal salts, and organic or amino acids, such as arginine, in particular water-soluble organic acids having a low molecular weight, such as citric acid and lactic acid. Optionally, one of these compounds may be used in combination with a sweetener. For example, citric acid may be used in combination with saccharin sodium and/or xylitol in addition to sodium and magnesium salts.

Alternatively, organic solvents, such as ethanol, dexpanthenol and/or aromatic flavours, such as the ingredients of essential oils (menthol, thymol, cineol, myrtol) may be added to improve both the taste and the tolerability of these formulations. Furthermore, terpenes, such as cineol and myrtol, are known to have weak antimicrobial effects and are suggested to improve the ciliary beating frequency, thereby enhancing mucus clearance from the lower and upper respiratory tract.

The compositions of the invention may further include polymers, such as dextrans, hydroxypropylmethylcellulose (HPMC), chitosan, modified starches, etc., which may be useful to improve the tolerability of the formulation including taste and the adherence of the drug product to the surface cell layer, e.g. mucosa. Of these, chitosan is preferred since the antimicrobial efficiency of macrolides, such as azithromycin or clarithromycin alone or in combination may be enhanced. These polymeric compounds are deemed to improve the adherence and adhesion of the drug when administered topically and may support a slow release effect to reduce dosing frequency.

In another particularly preferred embodiment, macrolides, e.g. azithromycin, clarithromycin, or a combination thereof, are formulated with water-soluble sodium, ammonium, magnesium and calcium salts. However, the so-called calcium asthma hypothesis reduces the applicability of calcium ions for taste-masking of formulations for inhalation. This hypothesis says that an increase in the ionised calcium concentration in the cytosol results in the release of allergic reaction mediators such as histamine and prostaglandin D2 by mastocytes and basophiles, and acetylcholine by cholinergic nerve endings. This subsequently induces smooth muscle contraction. Clearly, this is considered as an unacceptable feature of calcium-containing formulations for inhalation and treatment of respiratory diseases. On the other hand, magnesium is known to inhibit calcium flowing into the cells, thereby preventing smooth muscle contractions. Additionally, some magnesium salts are known to have an anti-oxidizing effect on stressed tissues and cells. The magnitude of the effect depends on the counter ion, with magnesium gluconate being approximately three times more potent than magnesium sulphate or magnesium chloride.

Several water-soluble sodium, ammonium and magnesium salts have been evaluated, and found to improve the taste of dissolved azithromycin formulations for inhalation when aerosolized for instance with an eFlow™ electronic nebuliser. However, it was surprisingly found that only a few salts were capable to simultaneously stabilize the liquid azithromycin formulations during storage. These salts are sodium and magnesium gluconate, -aspartate, -citrate, -acetate, -lactate, -succinate, and -maleate. Other calcium and magnesium salts, such as magnesium sulphate and magnesium chloride could only improve the taste and did not have the positive effect on the stability of azithromycin in solution. Furthermore, extensive sedimentation was observed during storage of formulations containing calcium chloride.

Using generally accepted prediction models (as for example described in Martin A., Physical Pharmacy, 4th edition, 1993, Williams & Wilkins, Baltimore), the time period was calculated in which the concentration of azithromycin remains above 90% of the original concentration for a liquid azithromycin formulation with magnesium gluconate. The predictions were based on measured concentrations during storage of the formulation at 25°C, 40°C and 70°C. This method of accelerated testing of pharmaceutical stability based on the principles of chemical kinetics was demonstrated by Garret and Carper (J. Am. Pharm. Assoc., Sci. Ed. 44, 515, 1995). The reaction rates for the decomposition of a drug in solution at various elevated temperatures are obtained by plotting the logarithm of concentration against time. Subsequently, the slope and intercept of the linear relation obtained when plotting the logarithms of the specific rates of decomposition against the reciprocals of the absolute temperatures (Arrhenius plot) was used to predict the decomposition rate at 5°C. Surprisingly, it was found that the concentration of azithromycin in a solution with magnesium gluconate remains above 90% of the original concentration during approximately 5.75 years when stored at 5°C.

The molar ratio of the macrolide (e.g. azithromycin or clarithromycin) to the above specific salt(s) (i.e. the at least one salt selected from the group consisting of sodium gluconate, sodium aspartate, sodium acetate, sodium lactate, sodium succinate, sodium maleate, magnesium gluconate, magnesium aspartate, magnesium citrate, magnesium acetate, magnesium lactate, magnesium succinate, and magnesium maleate) is in the range of about 1 : 0.5 to about 1 : 100; preferably it is in the range of about 1 : 1 to about 1 : 10, for example about 1 : 1.5, about 1 : 2, about 1 : 5 or about 1 : 10. If a plurality of the above specific salts is used, the aforementioned ratio (macrolide : salts) is determined in terms of the total concentration of the specific salts used. Preferably, at least an equimolar amount of said salt(s) (relative to the amount of macrolide) is used (i.e., the molar ratio of said salt(s) to macrolide is at least 1). In many cases, an at least equimolar amount of said salt(s) (relative to the amount of macrolide) is most advantageous to achieve the desired effect of taste-masking.

It is not fully clear why exactly the combination of the said salt(s) with a further different taste-masking agent is so effective in improving both the organoleptic and stability properties of various macrolide solutions; however, the inventors have found a surprising degree of synergy between these agents.

The compositions of the invention achieve unexpected stabilization and taste masking of macrolides in aqueous solution. This makes the use of further stabilizing or complexing agents, such as cyclodextrins, unnecessary. Thus, in a preferred embodiment, the compositions of the invention are free of such further stabilizing or complexing agents, in particular, free of cyclodextrins.

The dispersed phase of the aerosol prepared from the compositions of the invention exhibits a mass median diameter (MMD) preferably from about 1 to about 6 µm and more preferably from about 2 to about 4.5 µm or from about 1.5 to about 4 µm. These values should be understood as MMD values as determined by laser diffraction. Various appropriate analytical apparatuses to determine the MMD are known and commercially available, such as the Malvern MasterSizer X™ or Malvern SprayTec^{™}. The geometric distribution of the aerosolised liquid particles or droplets may be determined simultaneously with the MMD. In some embodiments, also the geometrical standard deviation (GSD) which characterises the broadness of the size distribution of the aerosol particles is of significance.

The selection of the precise MMD within the above described range should take the target region or tissue of the aerosol into account. For example, the optimal droplet diameter will differ depending on whether oral or nasal inhalation is intended, and whether oropharyngeal, bronchial, pulmonary, nasal, and/or paranasal sinus delivery is focussed upon. Additionally, the age of the patients determines the most appropriate particle size for drug delivery to the lungs. It is evident that for the inhalation treatment of infants much smaller mean droplet sizes will be required (< 2.5 µm) than for adults (< 5 µm).

If the aerosol is intended for prevention or treatment of a disease or condition of the oropharynx or the nasal cavity via, for instance, a spray pump, the MMD should be larger than about 9 µm. For the treatment of the upper airways, in particular the sinunasal mucosa, osteomeatal complex, and paranasal cavities, an MMD in the region of 2 to 4 µm is particularly suitable. Furthermore, it is suggested that the optimal MMD, leading to the relatively largest aerosol deposition, also depends on individual factors, in particular the size of the nose and geometry of the paranasal sinuses and the ostia through which the aerosol reaches the sinuses. For example, the volume of the sinuses and the diameter of the ostia differ substantially between individuals. If the individual sinunasal anatomy or a physiological parameter derived from the sinunasal anatomy of a person to be treated with an aerosol is at least partially known, it may be possible to select a particular MMD for optimised sinunasal or sinus delivery. In some embodiments, the aerosol prepared according to the invention may have an MMD of about 2.5 to 4.5 µm, in others from about 3 to about 4 µm, or from about 2 to about 3.5 µm, respectively. In further embodiments, the MMD is approximately (i.e. ± 0.25 µm) 2.0 µm, 2.5 µm, 3.0 µm, 3.5 µm, 4.0 µm or 4.5 µm.

On the other hand, if the aerosol is intended for pulmonary delivery, it may exhibit an MMD in the range from about 2.0 to about 4.5 µm and a GSD in the range from about 1.2 to about 1.8. More preferably, the aerosol prepared according to the invention, if adapted for pulmonary delivery, has an MMD in the range from about 2 to about 4.5 and a GSD in the range from about 1.4 to about 1.6. It has been found that each of these sets of combinations is particularly useful to achieve a high local drug concentration in the lungs, including the bronchi and bronchioli, relative to the amount of drug which is aerosolised. In this context it must be considered that deep lung deposition requires smaller MMDs than deposition in the central airways and that for younger children smaller droplet sizes are needed.

The aerosol can be generated with any conventional aerosol generator, for example, a nebuliser. As used herein, nebulisers are devices capable of aerosolising liquids. Preferably, the nebuliser is selected from jet, ultrasonic, piezoelectric, jet collision, electrohydrodynamic, capillary force, perforated membrane, or perforated vibrating membrane nebulisers as described in more detail by Knoch and Keller (Expert Opin. Drug Deliv., 2005, 2 (2), 377-390). If the intended use is the delivery of the active agent to an affected (or potentially affected) site of the lower airways such as the bronchi or the deep lungs, it is particularly preferred that a piezoelectric, electro-hydrodynamic, or perforated membrane-type nebuliser is selected for generating the aerosol. Examples of suitable nebulisers include the Mystis™, I-Neb™, MicroAir™, Multisonic™, Respimate™, eFlow™, AeroNeb™, AeroNeb Pro™, and Aero Dose™ device families.

As used herein, an aerosol generator is a device or a combination of devices capable of generating and emitting an aerosol. According to the present invention, the device is capable of aerosolising a liquid material into a dispersed liquid phase. Typically, such device is referred to as a nebuliser. Depending on the type and model of the device, the aerosol generator of the invention may require or include a compressor. In other words, the term aerosol generator is used for the complete apparatus or assembly required to produce and emit an aerosol and to administer the aerosol to an animal or to a human patient. A particularly preferred aerosol generator for application of an aerosol in the upper respiratory tract is the combination of the PARI SINUS^{™} compressor and a jet nebuliser, whereas a modified eFlow^{™} electronic nebuliser making use of a perforated vibrating membrane to generate an aerosol is a preferred aerosol generator for delivery of the formulation to the lower respiratory tract.

Another particularly preferred novel paranasal delivery concept (i.e. for drug delivery to the upper respiratory tract) is based on the aerosol generation via a perforated vibrating membrane principle as known for eFlow^{™}, but in combination with a pulsation of about 30 - 60 Hz to facilitate and to improve paranasal drug delivery.

According to a further preference, the nebuliser is adapted to deliver the major fraction of the loaded dose of liquid composition as aerosol, such as at least about 40 wt.-% of the loaded liquid composition. More preferably, at least 60 wt.% of the liquid composition filled into the nebuliser is actually emitted from the device, which is best achieved by using a modern, optionally customised electronic nebuliser based on the vibrating perforated membrane design. According to another embodiment, at least about 40 wt.-% of the composition charged into the medication reservoir is aerosolised, or even at least about 50 wt.% or up to 95 wt.-%, when breath-actuated or controlled breathing modes are applied.

On the other hand, if the aerosol is to be delivered to the nasal or sinunasal cavities or regions, it is preferred that the nebuliser is capable of emitting a pulsating (or vibrating) aerosol. Aerosols generated by such modified jet or electronic nebulisers can reach sinunasal or paranasal cavities much better than when the aerosol is generated in a continuous mode. These nebulisers have a nose piece for directing the aerosol flow into the nose. If only one nostril is used for inhalation of the aerosol, the other nostril must be closed by a suitable restrictor. Furthermore, this type of nebulisers characteristically releases an aerosol with pulsating pressure. The pulsating pressure waves achieve a more intensive ventilation of the sinuses so that a concomitantly inhaled aerosol is better distributed in these cavities (W. Möller et al., "Visualization of Human Sinus Ventilation by Radioactive Krypton using the PARI SINUS Pulsation System", Proceedings Respiratory Drug Delivery Europe, April, 2007, p.1-4). Examples for such nebulisation devices are disclosed in DE 102 39 321 B3.

Whether adapted for pulmonary or sinunasal delivery, the nebuliser should preferably be selected or adapted to be capable of aerosolising a unit dose at a preferred output rate. A unit dose is here defined as a volume of the liquid composition comprising the effective amount of active compound designated to be administered during a single administration. Preferably, the nebuliser can deliver such a unit dose at a rate of at least about 0.1 ml/min or, assuming that the relative density of the composition will normally be around 1, at a rate of at least about 100 mg/min. More preferably, the nebuliser is capable of an output rate of at least about 0.15 ml/min or 150 mg/min, respectively. In further embodiments, the output rates of the nebuliser are at least about 0.2, 0.3, 0.4, 0.5, 0.6, 0.7, 0.8, 0.9 or I ml/min delivering an aerosol with an MMD in the range from about 2 to about 4 µm.

Furthermore, the output rate of the nebuliser should be selected to achieve a short nebulisation time of the liquid composition. Obviously, the nebulisation time will depend on the volume of the composition which is to be aerosolised and on the output rate. Preferably, the nebuliser should be selected or adapted to be capable of aerosolising a volume of the liquid composition comprising an effective dose of the active compound within not more than about 20 minutes. More preferably, the nebulisation time for a unit dose is not more than about 10 minutes. In a further embodiment, the nebuliser is selected or adapted to enable a nebulisation time per unit dose of not more than about 6 minutes, or not more than about 3 minutes. Presently most preferred is a nebulisation time in the range from about 0.5 to about 5 minutes.

The liquid composition comprises an effective single dose of the active compound, i.e. the macrolide, dissolved in a volume of preferably not more than about 10 ml, more preferably not more than about 5 ml and most preferably between 0.25 and 2.5 ml. Such an amount of the liquid composition can be nebulised in preferably less than about 10 min, more preferably in less than about 8 min and most preferably in less than about 5 min.

Typically, daily doses of azithromycin are 500 mg when applied parenterally or oral ly. In one aspect, the pharmaceutical formulations of the invention may be designed to deliver a single effective dose of 500 mg when a very strong antibiotic effect is deemed necessary. In a further aspect, the invention may provide a pharmaceutical composition where the delivered dose is reduced with maintenance of the microbial effect. Preferably, the effective single dose for an antimicrobial effect in the airways after inhalation may range, depending on the application regime, between about 50 and 250 mg. In case a nebulizer is placed in the tubing circuit of a ventilator system in an emergency unit, higher inhaled doses (up to for example 1 g) may be needed (e.g. for treatment of hospital and community acquired pneumonia (HAP and CAP) or ventilator associated pneumonia (VAP)).

In another embodiment, the pharmaceutical composition is used primarily to achieve an anti-asthmatic, anti-inflammatory or immunomodulatory effect where the delivered doses may be in a range of about 5 - 50 mg with the objective of having a dose below a certain antimicrobial threshold to avoid resistance formation. Most probably, much lower doses of about 1 - 10 mg will be needed for topical administration of the inventive azithromycin solutions into the oropharyngeal region and nose.

In a preferred embodiment, the liquid composition of the invention will be administered from a preservative free multidose vial fitted with a metering pump delivering about 50 - 150 µl per actuation. Thus, the range of possible doses is from about 1 up to about 500 mg azithromycin per one single application, depending on the administration route, dosing frequency and efficiency of the drug delivery system.

As will be understood by a person skilled in the art, some of the features and preferences with respect to the liquid composition, as disclosed herein-above, may also be applied to the dispersed phase of the aerosol generated therefrom and vice versa. In particular, the dispersed phase of the aerosol comprises - like the liquid composition - an active compound, more specifically a macrolide antibiotic, having a poor taste, and a poor aqueous stability.

The liquid composition exhibits a dynamic viscosity in the range from about 0.8 to about 3 mPa·s. The dynamic viscosity of the liquid composition has an influence on the droplet size distribution of the aerosol formed by nebulisation and on the efficiency of nebulisation. According to another preferred embodiment, the dynamic viscosity is in the range of about 1.0 to about 2.5 mPa·s.

According to another embodiment, where the compositions are used for topical application in the oropharyngeal region or the nose, the dynamic viscosity can be increased to values ranging between 1 and 100 mPa·s, preferably between 1 and 10 mPa·s, more preferably between 2 and 5 mPa·s, in order to increase the residence time of the formulation on the site of infection or inflammation. To this end, the composition can contain at least one polymeric excipient with bioadhesive properties and potentially tissue penetration enhancers, such as vitamin E-TPGS, cellulose derivatives, e.g. methyl or hydroxypropylmethyl cellulose, or dextrans, hydroxyethylstarch, polyvinylpyrrolidone, polyvinylalcohol or chitosans.

To obtain an aerosol which is highly suited for the preferred uses described herein, the surface tension of the liquid composition should preferably be adjusted to the range of about 25 to 80 mN/m, and more preferably to the range of about 30 to 75 mN/m. In this context, it is to be taken into consideration that, in the lowest part of this range, a particularly good adhesion and spreadability of the preparation on the mucous membranes may be expected, but that the quality of the aerosol and the efficiency of the nebulisation could be adversely affected. Surprisingly, it was found that the novel formulations may exhibit reduced surface tensions in the range of 30 - 65 mN/m although no surfactant was added.

On the other hand, if the incorporation of a surfactant appears necessary, e.g. for taste-masking reasons, it can hardly be avoided that the surface tension is reduced fairly markedly below that of water or physiological buffer solution. Thus, a compromise may have to be found in each case depending on the active compound and the intended application.

In order to be well-tolerated, an aerosol should, as far as possible, have a physiologic tonicity or osmolality. Thus, it may be desirable to incorporate an osmotically active excipient to control the osmolality of the liquid formulation. The content of this excipient (or excipients, if a combination of substances is used) should be selected to yield an osmolality of the liquid formulation which does not deviate too much from that of physiological fluids, i.e., from about 290 mOsmol/kg. Surprisingly, it was found that the novel formulations were tolerable even when osmolality was as high as 1500 mOs-mol/kg. Furthermore, it was observed that azithromycin formulations exhibiting drug concentrations of 75 - 100 mg/ml with osmolalities between 800 and 1200 mOsmol/kg induced mucus clearance, but did not cause uncontrolled coughing as known for hypertonic saline or mannitol solutions exceeding osmolalities of about 1000 mOsmol/kg. Hence, it was unexpectedly found that these hyperosmotic azithromycin formulations support a desired mucus and sputum clearance and thus additionally facilitate expectoration of bacteria localized and enriched in the sputum. These unexpected desirable features may offer a substantial therapeutic advantage over oral drug application.

Surprisingly, it was also found that the addition of magnesium and sodium salts as complexing and taste-masking agent improves the tolerability of azithromycin formulations upon inhalation via an eFlow™ nebuliser even when these formulations have an osmolality of up to 1500 mOsmol/kg.

It is further believed that for sinunasal delivery, an optimised aerosol osmolality may not be as critical as, for example, in the case of deep lung delivery of aerosols. Thus, the intended use of the aerosol should be taken into account when selecting the osmolality of the liquid composition. In general, an osmolality in the range of 600 up to 1200 mOsmol/kg may be acceptable for those azithromycin formulations containing magnesium salts for both taste-masking and stability improvement. In particular, an osmolality in the range of about 200 up to about 400 mOsmol/kg is preferred for formulations designed to be administered topically, for example in the nose or the oropharyngeal region.

Thus, the osmolality of the liquid composition of the invention is generally in the range of 150 mOsmol/kg to 1500 mOsmol/kg, preferably in the range of 300 mOs-mol/kg to 1200 mOsmol/kg.

Optionally, the liquid composition may comprise further pharmaceutically acceptable excipients, such as osmotic agents, in particular inorganic salts; excipients for adjusting or buffering the pH, such as organic or inorganic salts, acids, and bases; bulking agents and lyophilisation aids, such as sucrose, lactose, trehalose, mannitol, sorbitol, xylitol, and other sugar alcohols; stabilisers and antioxidants, such as vitamin E or vitamin E derivatives, such as Vitamin E-TPGS, lycopene and its derivatives, ascorbic acid, sulphites, hydrogen sulphites, gallic acid esters, butyl hydroxyanisole, and butyl hydroxytoluene.

In one of the preferred embodiments, one or more osmotic agents such as sodium chloride are incorporated in the composition to adjust the osmolality to a value in the preferred range as outlined herein-above. It was observed that the tolerability of inhaled formulations with high osmolalities was improved when the sodium chloride concentration was greater than 30 mmol. Sodium chloride may be either added or formed *in situ* due to a salt forming process.

According to another preference, the composition comprises at least one excipient to adjust the pH. In order to provide a well-tolerated aerosol, the preparation according to the invention should be adjusted to a euhydric pH-value. The term "euhydric" implies that there may be a difference between pharmaceutical and physiological requirements regarding pH. This means for example that the pH will often be a compromise between on the one hand the pH at which stability of the preparation is guaranteed during a sufficiently long storage time and on the other hand a physiologically well-tolerated pH. Preferably, the pH-value lies in the slightly acidic to neutral region, i.e., between pH-values of about 4 to about 8. It is to be noted that deviations towards a weakly acidic environment can be better tolerated than shifts of the pH-value into the alkaline region. Thus, the pH-value is preferably in the range of about 4.5 to about 7.5, more preferably in the range of about 6 to about 7.

For adjusting and, optionally, buffering the pH-value, physiologically acceptable acids, bases, salts, and combinations of these may be used. Suitable excipients for lowering the pH-value or for use as the acidic component in a buffer system are strong mineral acids, such as sulphuric acid and hydrochloric acid. Moreover, inorganic and organic acids of medium strength as well as acidic salts may be used, such as for example phosphoric acid, citric acid, tartaric acid, succinic acid, fumaric acid, methionine, acidic hydrogen phosphates with sodium or potassium, lactic acid, glucuronic acid etc. However, sulphuric acid and hydrochloric acid are most preferred. Suitable excipients for raising the pH-value or for use as the basic component in a buffer system are particularly mineral bases such as sodium hydroxide or other alkali, alkaline earth hydroxides and oxides such as, in particular, magnesium hydroxide and calcium hydroxide, ammonium hydroxide and basic ammonium salts such as ammonium acetate, as well as basic amino acids such as lysine, carbonates such as sodium or magnesium carbonate, sodium hydrogen carbonate, citrates such as sodium citrate etc.

In one of the embodiments, the liquid composition of the invention contains a buffer system consisting of two components, and one of the particularly preferred buffer systems contains citric acid and sodium citrate. Nevertheless, other buffering systems may also be suitable.

For pharmaceutical reasons, the chemical stabilisation of the composition with further additives may be indicated. This mainly depends on the kind of active agent contained in the composition. The most common degradation reactions of chemically defined active agents in aqueous preparations comprise, in particular, hydrolysis reactions and oxidation reactions. Hydrolysis reactions may be primarily limited by optimal pH adjustment. Examples of active agents which may be subject to oxidative attack are those agents that have olefinic, aldehyde, primary or secondary hydroxyl, ether, thioether, endiol, keto or amino groups. Therefore, in the case of such oxidation-sensitive active agents, the addition of an antioxidant, optionally in combination with a synergistic antioxidant, may be advisable or necessary.

Antioxidants are natural or synthetic substances which prevent or interrupt the oxidation of the active agents. These are primarily adjuvants which are themselves oxidisable or which act as reducing agents, such as, for example, tocopherol acetate, retinol derivatives, such as vitamin A, lycopene, reduced glutathione, catalase, peroxide dismutase, selenoic acid.

Synergistic antioxidants are those which do not directly act as reagents in oxidation processes, but which counteract oxidation by an indirect mechanism such as the complexation of metal ions that catalyse oxidation. Such antioxidants are for example ascorbic acid, sodium ascorbate and other salts and esters of ascorbic acid (for example, ascorbyl palmitate), fumaric acid and its salts, malic acid and its salts, selenoic acid and its salts, butyl hydroxyanisole, propyl gallate, and sulphites such as sodium metabisulfite. Citric acid and citrates, malic acid and its salts, and maltol (3-hydroxy-2-methyl-4H-pyran-4-one) may also act as chelating agents.

If the liquid composition of the invention is not sufficiently stable to provide a commercially acceptable shelf-life it may be possible to extend the shelf-life by making provision that the liquid composition is stored under refrigeration. Alternatively, a suitable commercial formulation may be designed as a solid composition which is reconstituted prior to use by addition of an aqueous solvent. Typically, a solid composition of a chemically unstable active compound has the potential of a longer shelf-life.

Depending on the manufacturing method of the solid composition, one or more additional excipients may be useful. For example, if the composition is prepared by freeze drying (lyophilisation) or spray drying, which are particularly preferred methods for preparing such solid composition according to the invention, it may be useful to incorporate at least one lyophilisation aid and/or bulking agent, such as a sugar or a sugar alcohol, in particular sucrose, fructose, glucose, trehalose, mannitol, sorbitol, isomalt, or xylitol.

The solid composition is further characterised by the fact that the portion of the solid composition comprising an effective amount of the active compound (i.e. a unit dose), is dissolvable or dispersible in an aqueous solvent having a volume of preferably not more than about 10 ml. In other embodiments, it is dissolvable or dispersible in an aqueous liquid volume of not more than about 5 ml, not more than about 4, or even not more than about 2 ml. In addition, nebulisation or inhalation takes less than 15 min and more preferably less than 8 minutes.

As defined herein, "dissolvable" means that the solid composition and the aqueous solvent can be combined to form a solution or colloidal solution, whereas the term "dispersible" indicates that liquid dispersions such as micro-suspensions are formed when combining the solid composition and the aqueous solvent.

The solid composition for reconstitution may be part of a pharmaceutical kit. Such kit preferably comprises the solid composition in sterile form. As used herein, the term "sterility" is to be defined according to the usual pharmaceutical meaning. It is understood as the absence of germs which are capable of reproduction. Sterility is determined with suitable tests which are defined in the relevant pharmacopoeias. According to current scientific standards, a sterility assurance level (SAL) of 10⁻⁶ is generally regarded as acceptable for sterile preparations, i.e., one unit in a million might be contaminated.

As mentioned above, the solid composition may be prepared by providing a liquid composition similar to the liquid composition to be aerosolised, which is subsequently dried, for example by lyophilisation or spray drying. In this case, similar does not implicate that the liquid composition from which the solid composition is prepared by drying has to comprise all ingredients of the ready-to-use liquid composition. For example, it might be possible to design the aqueous solvent for reconstitution so that it comprises one or more of the excipients. Also, it is not necessary that the concentrations of the ingredients are identical for these two liquid compositions. Alternatively, the solid composition for reconstitution may be prepared by providing the active ingredient and, optionally, at least one excipient in powder form, which are subsequently mixed to obtain a powder mixture.

The liquid composition may be contained in single dose blow-fill-seal vials with a volume of 0.1 - 10 ml or in multidose vials from 5 - 50 ml having a spray or dispensing function.

The liquid composition can be aerosolized via a nebuliser into the upper or lower respiratory tract. Generation and administration of the aerosol is preferably characterized by one or more of the following features: a total output rate of at least 0.1 ml/min, a mass median diameter of about 1.5 to about 6 µm, a geometric standard deviation of about 1.3 - 2.8, the dose exiting the mouthpiece is larger than 25% of the loaded drug dose, and more than 50% of the emitted drug content is contained in droplets < 5 µm.

Preferably, the composition is administered using a regimen of repeated administration over a course of at least about five days. Optionally, the duration of the regimen is at least about one week, or about 10 days or about 2 weeks. In further embodiments, the duration is in the range of months or years. Furthermore, the regimen preferably comprises once, twice or thrice daily applications or inhalation; most preferred is once or twice daily administration over the course of therapy. Other preferred regimen are once or twice a week. Topical application regimes in the nose or the oropharyngeal region may be comparable as outlined above.

As indicated above, the invention also provides a method of generating an aerosol, said method comprising: (a) providing a liquid aqueous composition of the invention; (b) providing an aerosol generator capable of aerosolising the composition; and (c) operating said aerosol generator to aerosolise the composition. The method may further comprise a step of delivering the aerosol into the upper or lower respiratory tract of a human or animal.

The following examples serve to illustrate the invention; however, these are not to be understood as restricting the scope of the invention.

### EXAMPLES

### Example 1

54.3 g of azithromycin monohydrate ethanolate (corresponding to 50.0 g azithromycin) was dispersed in approximately 600 ml water for injection containing 20.0 g xylitol, 0.25 g saccharin sodium, and 0.25 g levomenthol. To dissolve azithromycin, the solution was acidified by dropwise addition of 2 N HCl under continuous stirring, until a clear solution was obtained. Immediately after dissolving azithromycin, 36.6 g magnesium gluconate dihydrate was added to the solution (molar ratio of azithromycin : magnesium gluconate was 1 : 1.2). This resulted in a solution with a pH of 5.2, which was adjusted to 6.3 by dropwise addition of 1 N NaOH. Water for injection was added to the solution to obtain a total volume of 1000 ml. Subsequently, the solution was sterile filtered under laminar air flow by using a 0.22 µm sterile filter and 8 ml were filled in presterilized amber glass vials. The osmolality of the solution was 738 mOsmol/kg. The solution tasted less bitter than a similar azithromycin solution without magnesium gluconate.

### Example 2

Similar as described in Example 1, a 7.5% (w/v) solution of azithromycin monohydrate ethanolate was prepared. In this case, 8.14 g of azithromycin monohydrate ethanolate (corresponding to 7.50 g azithromycin) was dispersed in approximately 60 ml water for injection containing 2.0 g xylitol, 0.045 g saccharin sodium, and 0.03 g levomenthol. As in Example 1, azithromycin was dissolved by dropwise addition of 2 N HCl and magnesium gluconate monohydrate was added to the solution (molar ratio of azithromycin : magnesium gluconate monohydrate was 1 : 1.05, corresponding to the addition of 4.55 g magnesium gluconate monohydrate). The pH was adjusted to 6.3 with 1 N NaOH and water for injection was added to obtain a total volume of 100 ml. Subsequently, 2 ml of the solution was sterile filtered under laminar air flow in pre-sterilized blow-fill-seal vials containing sterile nitrogen gas. The dynamic viscosity of the solution was 1.72 mPa·s and osmolality was 810 mOsmol/kg. Upon nebulisation by a PARI eFlow^{™} 30L electronic nebuliser and inhalation of this solution by 8 volunteers only a slightly bitter taste could be identified whereas a solution without magnesium gluconate tasted very unpleasant and bitter. Furthermore, the formulation was not irritating and no cough was induced.

### Example 3

A similar formulation as described in Example 2 was prepared with azithromycin dihydrate instead of azithromycin monohydrate ethanolate. Here, 7.86 g azithromycin dihydrate (corresponding to 7.50 g azithromycin) was dissolved in 100 ml water for injection containing 2.0 g xylitol, 0.045 g saccharin sodium, 0.03 g levomenthol, and 4.55 g magnesium gluconate monohydrate. The pH was adjusted to 6.3. The method for preparing the solutions was the same as described for Example 2. The azithromycin dihydrate solution had a dynamic viscosity of 1.70 mPa·s and an osmolality of 777 mOsmol/kg. Again, the bitter taste of azithromycin was masked well and inhalation did not cause the bad taste sensation and the intolerability that was experienced when inhaling an azithromycin solution without magnesium gluconate.

### Example 4

The nebulisation efficiency of the formulation described in Example 3 with the PARI eFlow^{™} nebuliser was investigated by breath simulation and laser diffraction. Breath simulation experiments were performed using a COMPAS™ breath simulator (PARI GmbH, Starnberg, Germany). A standard adult sinusoidal breathing pattern with 500 ml tidal volume, 15 breaths per minute and an inspiration to expiration ratio of 1 : 1 was applied. The device was filled with 1 ml of the formulation and connected via a filter to the breath simulator. The nebuliser was operated until it switched off automatically. Azithromycin collected on the inhalation filter was recovered and analyzed by a validated High Pressure Liquid Chromatographic (HPLC) method and UV detection to quantify the delivered dose. Assessment of the geometric droplet size distribution of the aerosol was conducted by laser diffraction using a Malvern MasterSizerX™. The aerosol was measured at a flow rate of 20 l/min entrained air, conditioned to 23°C and 50% relative humidity.

The aerosol produced by the eFlow^{™} nebuliser had a mass median diameter (MMD) of 3.6 µm with 75% of the droplets being smaller than 5 µm. Upon nebulisation, 72% of the initially charged azithromycin amount was found on the inspiratory filter. The mean nebulisation time (n=3) for 1 ml of the azithromycin solution (75 mg/ml) was between 2.4 and 2.5 min.

### Example 5

In another nebulisation experiment, a sample of 2 ml of the azithromycin solution of Example 3 was tested for its sinunasal aerosol delivery efficiency using a PARI SINUS nasal/paranasal drug delivery system (providing pressurised air which pulsates at a frequency of 44 Hz) for nebulisation of the aerosol into a human sinunasal cast model. The cast model is equipped with two cavities (representing the sinuses) in frontal, maxillary and sphenoid position. Cavities as well as orifices (ostia) are exchangeable, allowing variation of the sinus volume (7.5, 13 and 23 ml) and ostium diameter (0.5, 1.0 and 2.0 mm).

In this experiment, the model was equipped with 0.5 mm / 7.5 ml cavities in frontal, 1.0 mm / 13 ml cavities in sphenoid and 2.0 mm / 23 ml sinuses in maxillary position. Ostium length was 10 mm for all diameters. Filter pad liners were inserted into the sinus flasks in order to improve reproducibility of deposition.

The sample was nebulised for 8 minutes, i.e. 4 minutes in each nostril. After the experiment, all parts of the experimental set-up that were in contact with the inhalation solution, the cavities with ostia, the model, the nebuliser and the expiratory filter, were extracted with a defined volume of solvent. The nebuliser was weighed before and after the experiment for the gravimetric determination of the aerosol output.

In result, the sinus deposition was 7% of the loaded azithromycin content and the nasal cavity deposition was 10% of the loaded drug content. The emitted drug percentage was 26%.

### Example 6

In a further example, the influence of different magnesium and sodium salts on taste-masking and stability of azithromycin solutions was evaluated. In this screening, 6 aliquots of a solution containing 10% (w/v) azithromycin (in the form of azithromycin monohydrate ethanolate), 2.0% (w/v) xylitol, 0.03% (w/v) saccharin sodium, and 0.02% (w/v) levomenthol were prepared, according to the method described in Examples 1 to 3. To each of the aliquots another magnesium or sodium salt was added and the molar ratio of azithromycin to the salt was I : 1. The type and concentration of the different salts are summarised in Table 1. Formulations 4, 5, and 6 are for comparison.

**Table 1. Concentration of magnesium and sodium salts used in taste-masking and stability screening**

| | **Type of salt** | **Concentration % (w/v)** |
|---|---|---|
| **Formulation 1** | Magnesium gluconate dihydrate | 6.18 |
| **Formulation 2** | Magnesium aspartate dihydrate | 2.57 |
| **Formulation 3** | Magnesium hydrogen citrate | 3.10 |
| **Formulation 4** | Magnesium chloride hexahydrate | 2.72 |
| **Formulation 5** | Magnesium sulphate heptahydrate | 3.29 |
| **Formulation 6** | Sodium chloride | 0.78 |

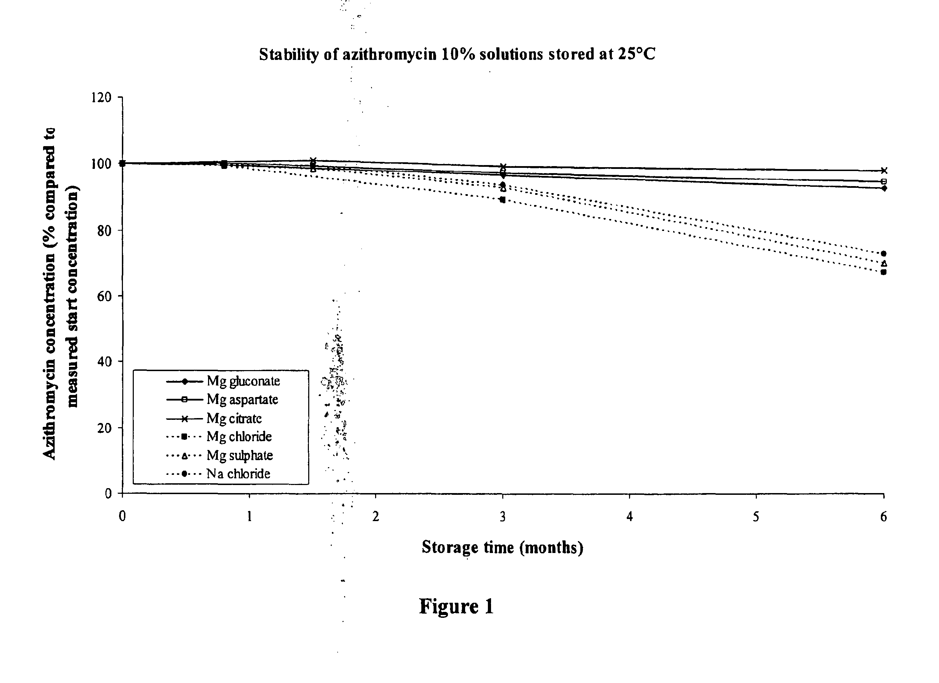

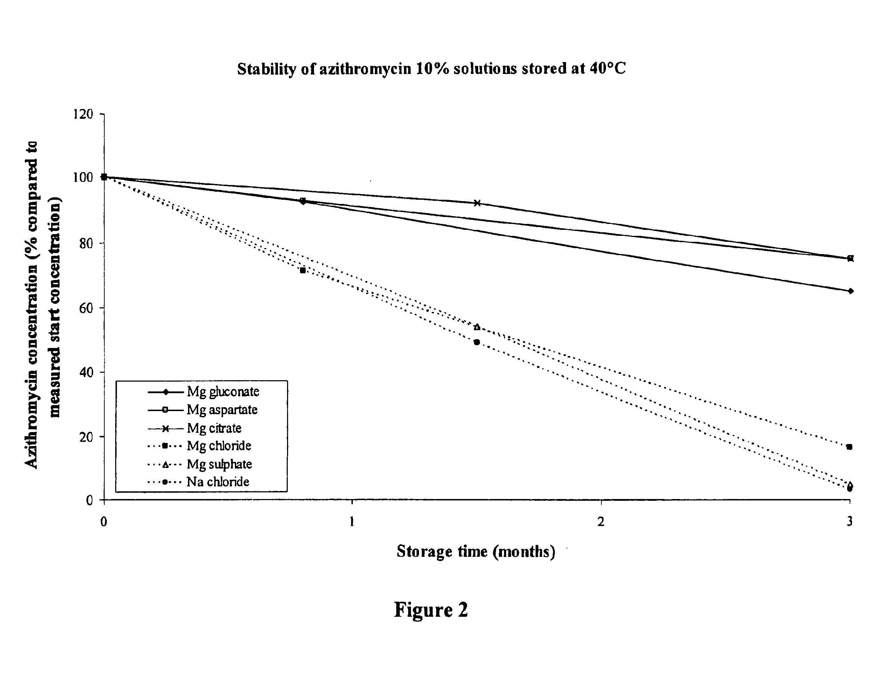

All formulations were sterile filtered under laminar air flow using a 0.22 µm sterile filter and 8 ml was filled in sterile amber glass vials. The vials were stored at three different temperatures, being 4 - 6°C (fridge), 25°C (60% RH), and 40 °C (75% RH). The azithromycin concentration was quantified by a sensitive High Pressure Liquid Chromatographic (HPLC) method with UV detection at 215 nm immediately after production, and after 3 or 6 weeks, 3 months and 6 months storage. The results are summarised in Table 2 and shown graphically in Figures 1 and 2. All solutions remained clear during storage. It was found that all magnesium salts were capable of masking the bitter taste of azithromycin, whereas this was not possible when using sodium chloride. Surprisingly, it was found that only magnesium gluconate, magnesium aspartate, and magnesium hydrogen citrate were capable to reduce the degradation of azithromycin in solution when stored at 25°C and 40°C as apparent from Figures 1 and 2, respectively.

**Table 2. Influence of magnesium or sodium salt on the concentration of azithromycin in solution when stored at different temperatures for different time periods**

| | **Storage time*** | **Azithromycin concentration (%, compared to initially measured concentration)** | | |
|---|---|---|---|---|
| | | **4 - 6°C (fridge)** | **25°C / 60% RH** | **40°C / 70% RH** |
| **Mg gluconate** | **3 w** | 100.9 | 99.5 | 92.2 |
| | **3 m** | 100.6 | 96.4 | 64.6 |
| | **6 m** | 101.3 | 92.4 | n/a |
| **Mg aspartate** | **3 w** | 100.9 | 100.0 | 92.3 |
| | **3 m** | 100.5 | 97.1 | 74.5 |
| | **6 m** | 100.6 | 94.3 | n/a |
| **Mg citrate** | **6 w** | 102.3 | 100.8 | 91.7 |
| | **3 m** | 101.9 | 99.0 | 74.6 |
| | **6 m** | 101.1 | 97.5 | n/a |
| **Mg chloride **** | **3 w** | 100.6 | 99.4 | 71.2 |
| | **3 m** | 100.6 | 89.0 | 16.4 |
| | **6 m** | 101.8 | 67.3 | n/a |
| **Mg sulphate **** | **6 w** | 99.8 | 98.3 | 53.9 |
| | **3 m** | 99.7 | 92.7 | 4.8 |
| | **6 m** | 99.6 | 70.0 | n/a |
| **NaCl**** | **6 w** | 100.8 | 99.3 | 48.9 |
| | **3 m** | 100.1 | 93.5 | 3.1 |
| | **6 m** | 99.4 | 72.8 | n/a |

| | | | | |
|---|---|---|---|---|
| * w = weeks; m = months. ** for comparison | | | | |

### Example 7

A solution of erythromycin was prepared with and without magnesium gluconate, where the latter formulation serves as a reference for evaluation of the taste-masking effect of magnesium gluconate:

A first solution was prepared by dissolving 0.03 g levomenthol, 0.045 g saccharin sodium and 2.0 g xylitol in approximately 60 ml water for injection. Afterwards, 7.50 g erythromycin was dispersed and dissolved by dropwise addition of 2 N HCl. Immediately after obtaining a clear solution, 4.64 g magnesium gluconate monohydrate (molar ratio of erythromycin to the magnesium salt was 1 : 1.05) was added and dissolved under continuous stirring. The pH of the obtained solution was adjusted to pH 8.0 by dropwise addition of 1 N NaOH and the volume was adjusted to 100 ml with water for injection.

A second solution was prepared as described for the first solution, with the exception that no magnesium gluconate was added. Both solutions were sterile filtered under laminar air flow with a 0.22 µm sterile filter and filled in sterile amber glass vials.

The osmolality of the formulation containing magnesium gluconate was 865 mOsmol/kg, whereas the osmolality was 536 mOsmol/ml for the formulation without magnesium gluconate. The taste of erythromycin was better masked in the solution containing magnesium gluconate compared to the solution without magnesium gluconate.

### Examples 8

The stability of azithromycin dihydrate solutions (7.5% (w/v) azithromycin) at different pH values was evaluated. Therefore, four 1000 ml aliquots of the solution described under Example 3 were prepared. The different aliquots were adjusted to different pH values, being 5.8, 6.3, 6.8 and 7.3, by dropwise addition of 1 N NaOH. The solutions were sterile filtered under laminar air flow by using 0.22 µm sterile filters and divided in aliquots of approximately 8 ml in sterile amber glass vials. Immediately after formulating the solutions, the dynamic viscosity and osmolality were measured. Dynamic viscosity was 1.70, 1.70, 1.66 and 1.65 for the formulations with pH 5.8, 6.3, 6.8 and 7.3, respectively. The osmolality of these solutions was 779, 777, 784 and 787 mOsmol/kg, respectively. The vials were stored at 4 - 6°C (fridge), 25°C (60% RH), and 40°C (75% RH). The azithromycin concentration was evaluated after 1.5, 3, 6 and 12 months storage and related to the concentration measured immediately after preparing the solutions. Results are shown in Table 3.

**Table 3. Influence of initial pH on the concentration of azithromycin dihydrate in solution when stored at different temperatures**

| **Target pH** | **Storage time (months)** | **Azithromycin concentretion (%, compared to initially measured concentration)** | | |
|---|---|---|---|---|
| | | **4 - 6°C (fridge)** | **25°C / 60% RH** | **40°C / 70% RH** |
| 5.8 | 1.5 | 99.9 | 98.2 | 83.5 |
| | 3 | 98.7 | 95.3 | 58.9 |
| | 6 | 98.2 | 91.2 | 27.0 |
| | 12 | 98.1 | 80.7 | n.a. |
| 6.3 | 1.5 | 99.2 | 98.3 | 84.0 |
| | 3 | 98.1 | 95.3 | 60.2 |
| | 6 | 98.0 | 91.8 | 27.7 |
| | 12 | 97.5 | 81.1 | n.a. |
| 6.8 | 1.5 | 98.9 | 97.1 | 84.7 |
| | 3 | 99.0 | 95.5 | 62.7 |
| | 6 | 97.7 | 92.2 | 31.0 |
| | 12 | 97.3 | 82.0 | n.a. |
| 7.3 | 1.5 | 98.3 | 95.4 | 84.5 |
| | 3 | 97.8 | 93.9 | 64.4 |
| | 6 | 97.3 | 91.4 | 32.2 |
| | 12 | 96.1 | 82.0 | n.a. |

### Example 9

Similarly as in Example 8, the stability of azithromycin monohydrate ethanolate solutions (7.5% (w/v) azithromycin) at different pH values was evaluated. Therefore, four 1000 ml aliquots of the solution described under Example 2 were prepared. The different aliquots were adjusted to different pH values, being 5.8, 6.3, 6.8 and 7.3, by dropwise addition of 1 N NaOH. The solutions were sterile filtered under laminar air flow by using 0.22 pm sterile filters and divided in aliquots of approximately 8 ml in sterile amber glass vials. The vials were stored at 4 - 6°C (fridge), 25°C (60% RH), and 40°C (75% RH). Immediately after formulating the solutions, the dynamic viscosity and osmolality were measured. Dynamic viscosity was 1.74, 1.72, 1.75 and 1.72 for the formulations with pH 5.8, 6.3, 6.8 and 7.3, respectively. The osmolality of these solutions was 818, 810, 815 and 813 mOsmol/kg, respectively. The azithromycin concentration was evaluated after 1.5, 3 and 6 months storage. The results, i.e. the concentrations in relation to the initially measured concentration, are shown in Table 4.

**Table 4. Influence of initial pH on the concentration of azithromycin monohydrate ethanolate in solution when stored at different temperatures**

| **Target pH** | **Storage time (months)** | **Azithromycin concentration (%, compared to initially measured concentration)** | | |
|---|---|---|---|---|
| | | **4 - 6°C (fridge)** | **25°C / 60% RH** | **40°C / 70% RH** |
| 5.8 | 1.5 | 100.9 | 100.4 | 82.5 |
| | 3 | 101.9 | 94.4 | 56.5 |
| | 6 | 102.4 | 93.7 | 25.1 |
| 6.3 | 1.5 | 102.1 | 100.8 | 84.5 |
| | 3 | 101.5 | 98.6 | 60.1 |
| | 6 | 103.1 | 95.6 | 26.7 |
| 6.8 | 1.5 | 102.8 | 100.6 | 86.3 |
| | 3 | 100.2 | 97.8 | 63.0 |
| | 6 | 101.6 | 96.3 | 29.7 |
| 7.3 | 1.5 | 100.9 | 97.9 | 86.0 |
| | 3 | 99.8 | 95.4 | 64.6 |
| | 6 | 101.3 | 94.1 | 30.5 |

### Example 10

32.24 g azithromycin dihydrate was dispersed in 300 ml water for injection. Azithromycin was dissolved by dropwise addition of 2 N HCl and after obtaining a clear solution, the pH was adjusted to 6.3 with 1 N NaOH. Subsequently, water for injection was added to obtain a total volume of 400 ml, which resulted in a solution with 7.5% (w/v) azithromycin. After that, 20 ml aliquots of this solution were filled in 25 ml volumetric flasks. Magnesium gluconate monohydrate (ratio of azithromycin : magnesium gluconate = 1 : 1.05, corresponding to 4.55% (w/v) magnesium gluconate monohydrate) was added to half of the samples, which were stirred until a clear solution was obtained. Additionally, different excipients were added to the solutions in different concentrations, according to the scheme in Table 5. Formulation 1 is for comparison.

Subsequently, the total volume of each aliquot was increased to 25 ml with the initially prepared azithromycin solution. The resulting solutions were sterile filtered under laminar air flow using a 0.22 µm sterile filter. The solutions were divided in 3 ml aliquots in 6 ml glass vials and lyophilized in a Christ LPC-16/NT Epsilon 2-6D freeze dryer. The freeze drying protocol was as follows: freezing for 2.5 h at -40°C, primary drying for 30 h at -35°C and 0.100 mbar followed by 6 h at -154°C and 0.08 mbar, and secondary drying for 10 h at 20°C and 0.009 mbar.

**Table 5. Excipients added to different azithromycin dihydrate solutions for freeze drying**

| **Form. Nr.** | **Mg gluconate (%)** | **Lactose (%)** | **Sucrose (%)** | **Xylitol (%)** |
|---|---|---|---|---|
| **1** | - | - | - | - |
| **2** | - | 2.50 | - | - |
| **3** | - | 7.50 | - | - |
| **4** | - | - | 2.50 | - |
| **5** | - | - | 7.50 | - |
| **6** | - | - | - | 2.00 |
| **7** | 4.55 | - | - | - |
| **8** | 4.55 | 2.50 | - | - |
| **9** | 4.55 | 7.50 | - | - |
| **10** | 4.55 | - | 2.50 | - |
| **11** | 4.55 | - | 7.50 | - |
| **12** | 4.55 | - | - | 2.00 |

All formulations yielded voluminous cakes after freeze drying. However, the cakes seemed less porous when the concentration of the sugar or sugar alcohol increased. The samples were reconstituted with water for injection, and all cakes dissolved within 1 minute. However, it was found that the samples containing magnesium gluconate dissolved somewhat slower than their counterparts without magnesium gluconate and that an increasing sugar concentration led to a longer reconstitution time.

After reconstitution, pH and osmolality were measured. Additionally, the taste of the formulations was given a score of 1, 2 or 3, corresponding to masked bitterness, slightly bitter or bitter, respectively. Furthermore, the freeze-dried samples were stored at 25°C during 6 weeks, after which they were reconstituted for measurement of the azithromycin concentration (expressed as the percentage of the azithromycin concentration in the solutions before freeze drying). The results of these evaluations, organised per formulation number as described in Table 5, are shown in Table 6. The addition of magnesium gluconate increased the stability of azithromycin in sugar containing formulations.

**Table 6. Evaluation of azithromycin dihydrate solutions after reconstitution of the freeze-dried cakes**

| **Form. Nr.** | **pH^{(a)}** | **Osmolality^{(a)} (mOsmol/kg)** | **Azithromycin concentration^{(b)} (%, compared to initially measured concentration)** | **Taste score^{(a)}** |
|---|---|---|---|---|
| **1** | n/a | n/a | 100.7 | 1 3 |
| **2** | 6.34 | 379 | 82.3 | 2 |
| **3** | 6.12 | 525 | 91.0 | 2 |
| **4** | 6.42 | 353 | 94.7 | 2 |
| **5** | 6.32 | 497 | 92.4 | 2 |
| **6** | 6.30 | 415 | 95.4 | 2 |
| **7** | n/a | n/a | 97.8 | 2 |
| **8** | 6.68 | 598 | 99.8 | 1 |
| **9** | 6.52 | 768 | 99.3 | 1 |
| **10** | 6.63 | 583 | 102.0 | 1 |
| **11** | 6.61 | 767 | 99.8 | 1 |
| **12** | 6.65 | 682 | 98.7 | 1 |

| | | | | |
|---|---|---|---|---|
| ^{(a)} Sample reconstituted immediately after drying ^{(b)} Sample reconstituted after 6 weeks storage of the lyophilised formulation at 25°C. | | | | |

### Example 11

A combination product of azithromycin dihydrate with tobramycin was formulated as follows: 1.069 g of azithromycin dihydrate was dispersed in approximately 80 ml water for injection containing 2.0 g xylitol, 0.045 g saccharin sodium, and 0.03 g levomenthol. 2 N HCl was added dropwise until all azithromycin dissolved and a clear solution was obtained. Thereafter, 0.606 g of magnesium gluconate monohydrate was added and the solution was stirred until a clear solution was obtained. Subsequently, 1.000 g of tobramycin was added to the azithromycin solution. This increased the pH value to approximately 9, which was readjusted to 6.3 by dropwise addition of 2 N HCl. Again, a clear solution was obtained and the total volume was increased to 100 ml by addition of water for injection, resulting in a solution containing 1.0% (w/v) azithromycin and 1.0% (w/v) tobramycin. The solution was sterile filtered under laminar air flow using a 0.22 µm sterile filter in sterile amber glass vials.

### Example 12

Similarly as described under Example 11, azithromycin dihydrate was combined with levofloxacin. The same amount of sucrose, saccharin sodium, levomenthol, azithromycin, and magnesium gluconate monohydrate was dissolved according to the method described in Example 11, and 1.000 g of levofloxacin was added after obtaining a clear solution. Here, pH only increased to about 6.8 and was adjusted to 6.3 by addition of a few drops of 2 N HCl. The volume was adjusted to 100 ml with water for injection, by which a solution containing 1.0% (w/v) azithromycin and 1.0% (w/v) levofloxacin was obtained. The formulation was sterile filtered under laminar air flow as described under Example 11.

### Example 13

Furthermore, a combined formulation containing 1.069 g azithromycin dihydrate and 1.000 g aztreonam was prepared in 100 ml of water for injection according to the method described in Examples 11 and 12 i.e. by dissolving mannitol, saccharin sodium and levomenthol in water for injection, adding azithromycin and dissolving the azithromycin by addition of HCl followed by addition of magnesium gluconate monohydrate, addition of aztreonam and adjusting of pH to 6.3. The resulting solution contained 1.0% (w/v) azithromycin and 1.0% (w/v) aztreonam. The formulation was sterile filtered under laminar air flow as described under Example 11.

### Example 14

The formulations prepared according to Examples 11, 12 and 13, were used to evaluate the Minimal Inhibitory Concentration (MIC) required to inhibit the growth of the following organisms: *Burkholderia cepacia, Haemophilus influenzae,* and *Pseudomonas aeruginosa,* using the agar dilution method based on DIN 58940 and 58944 according to NCCLS (National Committee on Clinical Laboratory Standards) criteria. As a reference, the MIC was also evaluated for formulations containing only azithromycin (1.0% w/v), tobramycin (10.0% w/v), or levofloxacin (2.0% w/v).

To determine MIC, Petri dishes containing Mueller-Hinton agar supplemented with agar-agar, or chocolate agar were prepared. Dilutions of the formulations prepared under Examples 11, 12, 13 and 14 were prepared in aqua purificata, resulting in solutions containing 1000 µg drug per ml (with exception of the formulation only containing levofloxacin, where a dilution containing 640 µg/ml was prepared). With these solutions, 2-fold dilution series were prepared in aqua purificata. After adding these dilutions to the liquid agar, an additional 10-fold dilution occurred. Two agar plates were poured for each test concentration and culture medium. After solidification and drying, the agar plates were inoculated with the test organisms and incubated as described in Table 7.

**Table 7. Inoculation and incubation**

| **Test organism** | | **CFU*** | **Growth conditions** | **Nutrient medium** | **Incubation** |
|---|---|---|---|---|---|
| ***Burkholderia cepacia*** | ATCC 17759 | 2.6 × 10⁷ | aerobic aero | Mueller-Hinton agar | 16 - 20 h at 36°C |
| ***Haemophilus influenzae*** | ATCC 49247 | 3.0 × 16⁷ | microaerophile | Chocolat agar | 16 - 20 h at 36°C |
| ***Pseudomonas aeruginosa*** | ATCC 9027 | 2.8 × 10⁷ | aerobic | Mueller-Hinton agar | 16 - 20 h at 36°C |

| | | | | | |
|---|---|---|---|---|---|
| * CFU = Colony Forming Units | | | | | |

The results of the tests are shown in Table 8. The MIC was given as the lowest concentration of the active substance at which there was no macroscopically visible growth. Minimal, barely visible growth or few small individual colonies were evaluated as inhibition. The MIC readings are generally subject to an error of up to two dilutions. According to the NICCLS, the following guidelines for sensitivity of azithromycin have been defined (breakpoints): 2 µg/ml is susceptible, 4 µg/ml is intermediate and ≥ 8 µg/ml is resistant. For *Haemophilus* spp., it was defined that a breakpoint ≤ 4 µg/ml is classified as susceptible. Inoculated control plates not containing active substances showed growth of the test organisms, whereas non-inoculated sterile control plates did not show microbial growth.

**Table 8. Results of the determination of MIC for different antibiotic formulations**

| **Test organism** | **MIC (µg/ml)** | | | | | |
|---|---|---|---|---|---|---|
| | **Azi.** | **Tobr.** | **Levo.** | **Azi. + Tobr.** | **Azi. + Levo.** | **Azi. + Aztr.** |
| ***Burkholderia cepacia*** | 25 | 50 | 4 | 6.25 | 6.25 | 12.5 |
| ***Haemophilus influenzae*** | 25 | 50 | 0.25 | 25 | 25 | 25 |
| ***Pseudomonas aeruginosa*** | 6.25 | 0.781 | 0.5 | 0.781 | 0.391 | 3.125 |

| | | | | | | |
|---|---|---|---|---|---|---|
| Azi. = azithromycin; Tobr. = tobramycin; Levo. = levofloxacin; Aztr. = aztreonam. | | | | | | |

It was shown that *Pseudomonas aeruginosa* was intermediately susceptible or susceptible to the different antibiotic formulations. The other two organisms were resistant to azithromycin and tobramycin, but intermediately susceptible to susceptible to levofloxacin and to the combination of azithromycin with either tobramycin or levofloxacin. Especially for *Burkholderia cepacia* tested with the first combination (azithromycin and tobramycin) this was surprising as, despite being resistant to azithromycin and to tobramycin, the susceptibility of the organism to the combined formulation could be classified as intermediate.

### Example 15

In a further example, a viscous azithromycin nose spray was prepared under laminar air flow, using sterilised powders. Firstly, 1 g of hydroxypropyl methylcellulose (HPMC) 4000 mPa·s was dissolved in 30 ml water for injection. Secondly, 1.069 g azithromycin dihydrate (corresponding to 1 g azithromycin) was dispersed in approximately 60 ml water for injection containing 2.0 g xylitol, 0.045 g saccharin sodium, and 0.03 g levomenthol, and dissolved by dropwise addition of 2 N HCl. Thereafter, 0.606 g magnesium gluconate (molar ratio of azithromycin : magnesium gluconate monohydrate is 1 : 1.05) was dissolved under continuous stirring and the pH of the solution was increased to 6.3 by dropwise addition of 1 N NaOH. This solution was then mixed with the HPMC solution and the volume was increased to a total of 100 ml with water for injection. The formulation was filled under laminar air flow into 0.5 ml pre-sterilized vials with a metering spray pump nozzle. The formulation showed pseudo-plastic behaviour and the dynamic viscosity measured at a shear rate of 300/s was 93.73 mPa·s, whereas the viscosity was 58.77 at a shear rate of 100/s.

### Example 16

A 1% azithromycin formulation was prepared by dispersing 1.069 g azithromycin dihydrate in approximately 60 ml water for injection containing 1 g trehalose, 0.045 g saccharin sodium, and 0.03 g levomenthol, and acidifying the solution by dropwise addition of 2 N HCl until a clear solution was obtained. Afterwards, 0.606 g magnesium gluconate (molar ratio of azithromycin : magnesium gluconate monohydrate is 1 : 1.05) was dissolved under continuous stirring and the pH of the solution was increased to 6.3 by dropwise addition of 1 N NaOH. After adjusting the total volume to 100 ml with water for injection, the solution was sterile filtered under laminar air flow with 0.22 µm sterile filters in sterile polyethylene bottles fitted with a metering spray pump nozzle, for administration of an aerosol mist in the oropharyngeal region. The azithromycin concentration was measured immediately after preparation, and after storage at 25°C and 40°C during 5 weeks. It was found that 98.1% of the originally measured azithromycin concentration was maintained when the formulation was stored at 25°C and 90.2% when stored at 40°C.

### Example 17

Two azithromycin dihydrate formulations were prepared, where the first formulation contained magnesium gluconate and the second formulation sodium gluconate. A solution containing 4.0 g xylitol, 0.09 g saccharin sodium and 0.06 g levomenthol in 120 ml water for injection was prepared. Subsequently, 15.721 g azithromycin dihydrate was dispersed and dissolved by dropwise addition of 2 N HCl. The solution was divided in 2 equal parts by weight. To the first part 4.55 g magnesium gluconate monohydrate (azithromycin : magnesium gluconate monohydrate ratio is I : 1.05) was added, whereas 2.29 g sodium gluconate was added to the second part of the solution. The solutions were stirred until the gluconate salts dissolved. Water for injection was added to each solution until a total volume of 100 ml was obtained. Both formulations were filtered under laminar air flow by use of 0.22 µm sterile filters in sterile amber glass vials. For both formulations, osmolality and dynamic viscosity were measured immediately after preparing the solutions. For azithromycin with magnesium gluconate, the results for these analyses were 777 mOsm/kg, and 1.7 mPa·s, respectively. For azithromycin combined with sodium gluconate, values of 701 mOsm/kg, and 1.56 mPa·s, respectively, were measured.

An accelerated stability test was performed by storing the formulations for 2 days at 70°C. The azithromycin concentration was measured and the percentage of recovered drug was calculated by comparing the concentration after storage with the concentration immediately after preparing the formulations. When azithromycin was combined with magnesium gluconate, 82.7 % was recovered. pH was found to be 5.71 and osmolality was 777 mOsmol/kg. When azithromycin was combined with sodium gluconate, 84.6 % was recovered, and the solution had a pH of 5.68 and an osmolality of 721 mOsmol/kg. Additionally, it was found that the bitter taste of azithromycin was well masked in both formulations.

### Example 18

In a further example, two formulations containing 2% (w/v) azithromycin monohydrate ethanolate in combination with citric acid and either calcium chloride or magnesium chloride were prepared. The calcium chloride containing formulation serves as a comparison to the claimed formulations. Azithromycin was dispersed and dissolved in approximately 60 ml water for injection containing 2.0 g xylitol, 0.045 g saccharin sodium and 0.03 g levomenthol by addition of anhydrous citric acid until a clear solution was obtained (0.515 g). Immediately thereafter, 0.4 g calcium chloride dihydrate (molar ratio of azithromycin : calcium chloride was I : 1) was added to the first solution (prepared for comparison), whereas 0.55 g magnesium chloride hexahydrate (molar ratio of azithromycin : magnesium chloride was 1 : 1) was added to the second azithromycin solution. The solutions were stirred until a clear solution was obtained. Afterwards, pH was adjusted to 5.3 by addition of dropwise 1N NaOH and the total volume of the solutions was increased to 100 ml with water for injection. The solutions were sterile filtered under laminar air flow with 0.22 µm sterile filters and filled in sterile amber glass vials. The vials were stored at 5°C during 1 year, after which flaky sediment was seen on the bottom of the vial when azithromycin was combined with calcium chloride, whereas the solution remained clear when azithromycin was combined with magnesium chloride.

### Example 19

10.86 g of azithromycin monohydrate ethanolate (corresponding to 10.0 g azithromycin) was dispersed in approximately 120 ml water for injection containing 4.0 g xylitol, 0.05 g saccharin sodium, and 0.05 g levomenthol. To dissolve azithromycin, the solution was acidified by dropwise addition of 2 N HCl under continuous stirring, until a clear solution was obtained. Immediately after dissolving azithromycin, 3.44 g magnesium acetate was added to the solution (molar ratio of azithromycin : magnesium acetate was 1 : 1.2). The pH was adjusted to 6.3 by dropwise addition of 1 N NaOH. Water for injection was added to the solution to obtain a total volume of 200 ml. Subsequently, the solution was sterile filtered under laminar air flow by using a 0.22 µm sterile filter and 8 ml were filled in pre-sterilized amber glass vials.

### Example 20

In this screening, 6 aliquots of a solution containing 7.5% (w/v) azithromycin (in the form of azithromycin dihydrate), 2.0% (w/v) xylitol, 0.045% (w/v) saccharin sodium, and 0.03% (w/v) cineol were prepared. Firstly, xylitol, saccharin sodium, and cineol were dissolved in water for injection. Afterwards, azithromycin was added and dissolved by dropwise addition of 2 N HCl under continuous stirring. A different magnesium or sodium salt was added to each of the aliquots (molar ratio of azithromycin to the cation was 1 : 1.05) and the pH was adjusted to 6.3 (with 1 N NaOH or HCl). The final volume of each aliquot was adjusted to 500 ml. The type and concentration of the different salts are summarised in Table 9.

**Table 9. Concentration of magnesium and sodium salts used in taste-masking and stability screening**

| | **Type of salt** | **Concentration % (w/v)** |
|---|---|---|
| **Formulation 1** | Magnesium acetate tetrahydrate | 2.26 |
| **Formulation 2** | Magnesium lactate hydrate | 2.51 |
| **Formulation 3** | Sodium gluconate | 2.30 |
| **Formulation 4** | Disodium succinate dihydrate | 0.85 |
| **Formulation 5** | Disodium maleate dihydrate | 1.03 |
| **Formulation 6** | Sodium aspartate monohydrate | 1.82 |

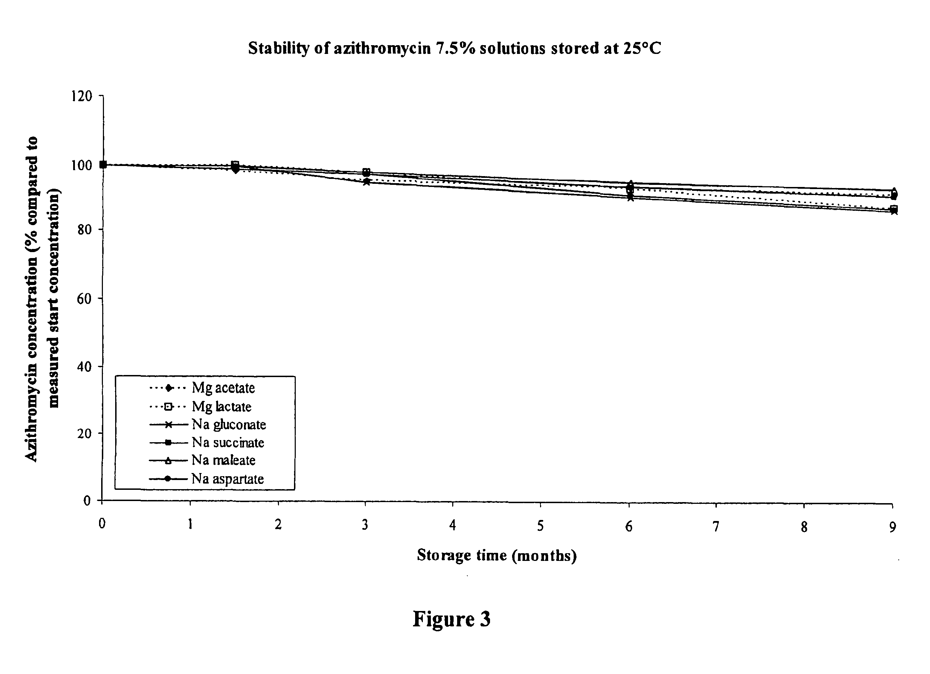

All formulations were sterile filtered under laminar air flow using a 0.22 µm sterile filter and 8 ml was filled in sterile amber glass vials. The vials were stored at three different conditions, being 4 - 6°C (fridge), 25°C (60% RH), and 40 °C (75% RH). The azithromycin concentration was quantified by a sensitive High Pressure Liquid Chromatographic (HPLC) method with UV detection at 215 nm immediately after production, and after 6 weeks, 3 months, 6 months and 9 months storage. The results are summarised in Table 10 and the 25°C storage data are shown graphically in Figure 3. All solutions remained clear during storage. Also these salts were capable of masking the bitter taste of azithromycin upon inhalation and to stabilise the aqueous formulation, especially when stored at 5°C.

**Table 10. Influence of magnesium or sodium salt on the concentration of azithromycin in solution when stored at different temperatures during increasing time periods**

| | **Storage time *** | **Azithromycin concentration (%, compared to initially measured concentration)** | | |
|---|---|---|---|---|
| | | **4 - 6°C (fridge)** | **25°C / 60% RH** | **40°C / 70% RH** |
| **Mg acetate** | **6 w** | 100.0 | 98.3 | 88.9 |
| | **3 m** | 99.1 | 95.2 | 71.7 |
| | **6 m** | 99.1 | 93.0 | 48.3 |
| | **9 m** | 99.3 | 91.0 | n.a. |
| **Mg lactate** | **6 w** | 101.2 | 99.9 | 85.7 |
| | **3 m** | 100.8 | 97.8 | 63.1 |
| | **6 m** | 100.9 | 92.6 | 25.1 |
| | **9 m** | 100.2 | 86.8 | n.a. |
| **Na gluconate** | **6 w** | 99.6 | 99.7 | 82.9 |
| | **3 m** | 98.2 | 94.7 | 56.8 |
| | **6 m** | 99.1 | 90.1 | 19.2 |
| | **9 m** | 99.4 | 85.8 | n.a. |
| **Na succinate** | **6 w** | 100.8 | 98.7 | 89.2 |
| | **3 m** | 101.2 | 97.2 | 75.2 |
| | **6 m** | 100.4 | 93.1 | 46.2 |
| | **9 m** | 99.5 | 90.5 | n.a. |
| **Na maleate** | **6 w** | 101.5 | 99.7 | 91.5 |
| | **3 m** | 100.9 | 97.7 | 80.2 |
| | **6 m** | 100.2 | 94.7 | 59.7 |
| | **9 m** | 101.0 | 92.4 | n.a. |
| **Na aspartate** | **6 w** | n.a. | 98.8 | 83.0 |
| | **3 m** | 99.5 | 97.2 | 51.0 |
| | **6 m** | 98.3 | 90.7 | 18.3 |
| | **9 m** | 98.6 | 86.5 | n.a. |

| | | | | |
|---|---|---|---|---|
| * w = weeks; m = months. | | | | |

### Example 21

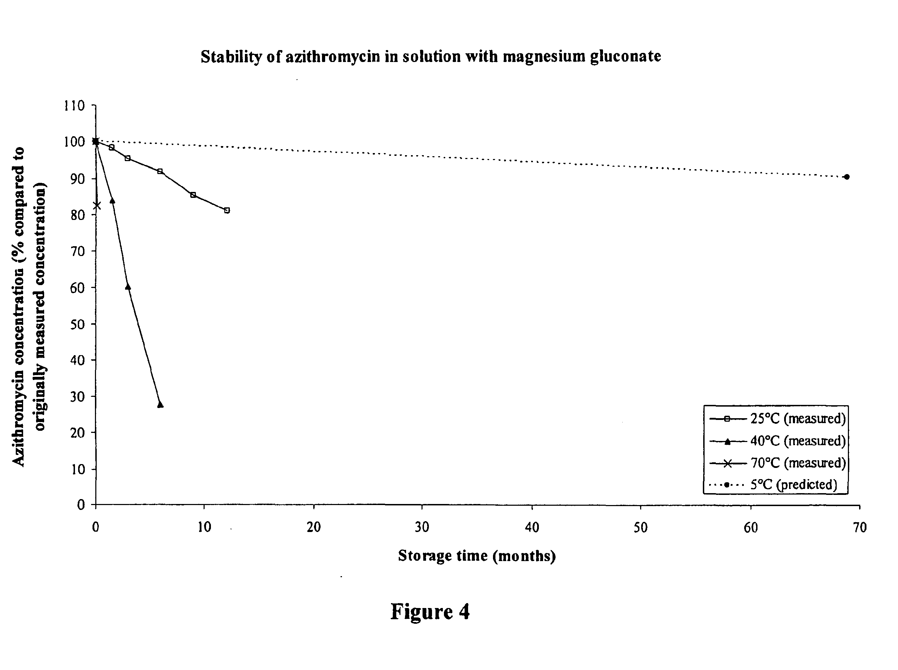

A 7.5% (w/v) solution of azithromycin monohydrate ethanolate was prepared. In this case, 40.70 g of azithromycin monohydrate ethanolate (corresponding to 37.50 g azithromycin) was dispersed in approximately 300 ml water for injection containing 10.0 g xylitol, 0.225 g saccharin sodium, and 0.15 g levomenthol. Azithromycin was dissolved by dropwise addition of 2 N HCl and 22.75 g magnesium gluconate monohydrate was added to the solution (molar ratio of azithromycin : magnesium gluconate monohydrate = I : 1.05). The pH was adjusted to 6.3 with 1 N NaOH and water for injection was added to a total volume of 500 ml. Subsequently, the solution was sterile filtered under laminar air flow in pre-sterilized amber glass vials. The vials were stored at 25°C, 40°C and 70°C. The azithromycin concentration was quantified by a sensitive High Pressure Liquid Chromatographic (HPLC) method with UV detection at 215 nm at different time points after storage. The concentration was measured after 1.5, 3, 6, 9 and 12 months storage at 25°C, after 1.5, 3 and 6 months when stored at 40°C, and after 2 days when stored at 70°C. The results are shown in Figure 4, and were used to predict the time period in which the concentration of azithromycin remains above 90% of the original concentration when stored at 5°C. The reaction rates for the decomposition of azithromycin at the different temperatures were obtained by plotting the natural logarithm of concentration against time. Subsequently, the slope and intercept of the linear relation obtained when plotting the natural logarithms of the rates of decomposition against the reciprocals of the absolute temperatures (Arrhenius plot) were used to predict the decomposition rate at 5°C. Surprisingly, it was found that the concentration of azithromycin in a solution with magnesium gluconate remains above 90% of the original concentration during approximately 5.75 years (69 months) when stored at 5°C (Figure 4).

### Example 22

In another example, clarithromycin solutions in combination with different magnesium and sodium salts have been prepared. Three aliquots of a solution containing 1.0% (w/v) clarithromycin, 0.3% (w/v) xylitol, and 0.01% (w/v) saccharin sodium were prepared in the same way as described above for azithromycin. After dissolving of clarithromycin by dropwise addition of 2 N HCl under continuous stirring, 0.61% (w/v) magnesium gluconate was added to the first aliquot, 0.31% (w/v) sodium gluconate was added to the second aliquot and 0.23% (w/v) magnesium citrate was added to the third aliquot. The pH was adjusted to 6.3 (with 1 N NaOH), and the volume of each aliquot was adjusted to 500 ml.

The formulations were sterile filtered in sterile amber glass vials and stored at 4 - 6°C (fridge), 25°C (60% RH), and 40 °C (75% RH). The clarithromycin content in the samples was measured after 6 weeks, 3 months, and 6 months storage. The results are shown in Table 11. The taste of the formulations upon inhalation was well-accepted.

**Table 11. Influence of magnesium or sodium salt on the concentration of clarithromycin in solution when stored at different temperatures during increasing time periods**

| | **Storage time *** | **Clarithromycin concentration (%, compared to initially measured concentration)** | | |
|---|---|---|---|---|
| | | **4 - 6°C (fridge)** | **25°C / 60% RH** | **40°C / 70% RH** |
| **Mg gluconate** | **6 w** | 98.9 | 99.7 | 95.1 |
| | **3 m** | 98.4 | 98.2 | 79.0 |
| | **6 m** | 99.0 | 96.5 | 56.3 |
| **Na gluconate** | **6 w** | 99.4 | 97.0 | 97.4 |
| | **3 m** | 103.4 | 97.5 | 98.0 |
| | **6 m** | 99.4 | 95.9 | 95.2 |
| **Mg citrate** | **6 w** | 99.5 | 97.4 | 94.5 |
| | **3 m** | 99.1 | 98.0 | 85.9 |
| | **6 m** | 98.8 | 95.2 | 65.0 |

| | | | | |
|---|---|---|---|---|
| * w = weeks; m = months. | | | | |

### Example 23

Two azithromycin dihydrate formulations were prepared, where the azithromycin : salt concentration ratio was 1 : 2. The salts were magnesium gluconate and sodium gluconate. The solutions further contained 0.045% (w/v) saccharin sodium, 0.03% (w/v) myrtol, and 2.0% (w/v) xylitol, and were prepared as described above. After adjusting pH to 6.3 and volume to 200 ml, the solutions were sterile filtered in sterile amber glass vials. These were stored at 25°C (60% RH) and 40 °C (75% RH), and analysed after 3 months storage. The azithromycin concentration (related to the measured start concentration) in the magnesium gluconate containing solutions was 95.9% and 58.0% when stored at 25°C and 40°C, respectively. When azithromycin was combined with sodium gluconate, 96.0% and 57.7% of the measured start concentration could be recovered after 3 months storage at 25°C and 40°C. These percentages are similar to the recovery when using an azithromycin : salt concentration ratio of 1 : 1.05.

### Example 24

A further solution containing 10.0% (w/v) azithromycin dihydrate was prepared where magnesium gluconate and magnesium citrate were combined to improve taste and stability of azithromycin. As described above, a solution containing 0.045% (w/v) saccharin sodium, 0.03% (w/v) cineol, and 2.0% (w/v) xylitol was prepared. Azithromycin dihydrate was added and dissolved by dropwise addition of 2 N HCl. Immediately thereafter, 2.88% (w/v) magnesium gluconate monohydrate and 1.55% (w/v) magnesium hydrogen citrate dihydrate were added. Subsequently, the pH of the solution was adjusted to 6.3. The formulation was inhaled with a PARI eFlow^{™} nebuliser. The taste was only marginally bitter and no irritation or cough-induction was noticed.

## Claims

1. A liquid aqueous pharmaceutical composition for administration as an aerosol to the respiratory tract, nose or oropharyngeal region comprising (i) a macrolide having a poor taste and poor chemical stability in aqueous solution; (ii) at least one salt selected from the group consisting of sodium gluconate, sodium aspartate, sodium acetate, sodium lactate, sodium succinate, sodium maleate, magnesium gluconate, magnesium aspartate, magnesium citrate, magnesium acetate, magnesium lactate, magnesium succinate, and magnesium maleate; or mixtures thereof and (iii) a taste-masking agent different from said salt; wherein
(a) the concentration of said macrolide in the composition is in the range of about 0.25 wt.-% to about 15 wt.-%;
(b) the molar ratio of said macrolide : said salt is in the range from about 1 : 0.5 to about 1 : 100;
(c) the pH of the composition is in the range of about 3 to 9; and
(d) the osmolality of the composition is in the range of about 150 mOsmol/kg to about 1500 mOsmol/kg.

2. The composition of claim 1, having a stability such that the concentration of the macrolide after storage for three years at 4°C to 8°C is at least 90% of the initial concentration.

3. The composition of claim 1 or 2, wherein the macrolide is azithromycin or clarithromycin or a pharmaceutically acceptable salt thereof.

4. The composition of any one of claims I to 3, wherein the salt is selected from the group consisting of sodium gluconate, magnesium gluconate, magnesium citrate, sodium succinate, sodium maleate, magnesium succinate, and magnesium maleate.

5. The composition of any one of claims 1 to 4, wherein the molar ratio of said macrolide : said salt is in the range from 1: 1 to 1: 10.

6. The composition of any one of claim 1 to 5, wherein the taste-masking agent is selected from the group consisting of sweeteners, sugars, and sugar alcohols.

7. The composition of any one of claims 1 to 5, wherein the taste-masking agent is selected from the group consisting of saccharin, saccharin sodium, aspartame, cyclamate, sucralose, acesulfame, acesulfame potassium, neotame, thaumatin, neohesperidine, neohesperidine dihydrochalcone, sucrose, trehalose, lactose, fructose, xylitol, mannitol, sorbitol, isomaltol, L-arginine, L-lysine, citric acid, lactic acid, cineol, myrtol, and levomenthol.

8. The composition of any one of claims 1 to 7, comprising a further drug substance selected from the group consisting of quinolons, aminoglycosides, peptide antibiotics, monobactams, cefalosporins, antifungals, immunmodulators, non-steroidal anti-inflammatory drugs, steroids, and pharmaceutically acceptable salts thereof.

9. The composition of any one of claims 1 to 8, wherein the composition is free of cyclodextrins.

10. A method of generating an aerosol, said method comprising:
(a) providing a composition according to any one of claims 1 to 9;
(b) providing an aerosol generator capable of aerosolising the composition; and
(c) operating said aerosol generator to aerosolise the composition.

11. The method of claim 10, wherein the aerosol generator is a vibrating and/or perforated vibrating membrane type nebuliser.

12. The method of claim 11, wherein the nebuliser is adapted for nasal or sinunasal administration and operated in a continuous, breath enhanced, breath triggered or pulsating mode.

13. The method of claim 10, wherein the composition contains a polymeric excipient with bioadhesive properties, exhibits a dynamic viscosity of about 1 mPa·s to about 10 mPa·s and an osmolality of about 200 mOsmol/kg to about 600 mOsmol/kg and the aerosol has a mass median droplet diameter of more than about 9 µm.

14. The use of the composition of any one of claims 1 to 9 for the manufacture of a medicament for the prophylaxis or treatment of diseases or conditions of the lower and upper respiratory tract, or infections or inflammation of the nose or oropharyngeal regions.

15. A solid pharmaceutical composition for preparing a liquid composition according to any one of claims 1 to 9, which solid composition comprises (i) a macrolide having a poor taste and poor chemical stability in aqueous solution; (ii) at least one salt selected from the group consisting of sodium gluconate, sodium aspartate, sodium acetate, sodium lactate, sodium succinate, sodium maleate, magnesium gluconate, magnesium aspartate, magnesium citrate, magnesium acetate, magnesium lactate, magnesium succinate, and magnesium maleate; and (iii) a taste-masking agent different from said salt.

## Patentansprüche

1. Wässrige flüssige pharmazeutische Zusammensetzung zur Verabreichung als ein Aerosol an den respiratorischen Trakt, die Nase oder die oropharyngeale Region, umfassend (i) ein Makrolid, das schlechten Geschmack und schlechte chemische Stabilität in wässriger Lösung hat; (ii) wenigstens ein Salz, ausgewählt aus der Gruppe, bestehend aus Natriumglukonat, Natriumaspartat, Natriumacetat, Natriumlactat, Natriumsuccinat, Natriummaleat, Magnesiumglukonat, Magnesiumaspartat, Magnesiumcitrat, Magnesiumacetat, Magnesiumlactat, Magnesiumsuccinat und Magnesiummaleat oder Gemischen davon, und (iii) ein von dem Salz verschiedenes Geschmacksmaskierungsmittel, wobei
(a) die Konzentration des Makrolids in der Zusammensetzung im Bereich von etwa 0,25 Gew.-% bis etwa 15 Gew.-% ist;
(b) das Molverhältnis des Makrolids: dem Salz im Bereich von etwa 1:0,5 bis etwa 1:100 ist;
(c) der pH der Zusammensetzung im Bereich von etwa 3 bis 9 ist, und
(d) die Osmolalität der Zusammensetzung im Bereich von etwa 150 mOsmol/kg bis etwa 1500 mOsmol/kg ist.

2. Zusammensetzung gemäß Anspruch 1, die eine solche Stabilität hat, dass die Konzentration des Makrolids nach Lagerung für drei Jahre bei 4°C bis 8°C wenigstens 90% der Anfangskonzentration ist.

3. Zusammensetzung gemäß Anspruch 1 oder 2, wobei das Makrolid Azithromycin oder Clarithromycin oder ein pharmazeutisch annehmbares Salz davon ist.

4. Zusammensetzung gemäß einem der Ansprüche 1 bis 3, wobei das Salz aus der Gruppe, bestehend aus Natriumglukonat, Magnesiumglukonat, Magnesiumcitrat, Natriumsuccinat, Natriummaleat, Magnesiumsuccinat und Magnesiummaleat, ausgewählt ist.

5. Zusammensetzung gemäß einem der Ansprüche 1 bis 4, wobei das Molverhältnis des Makrolids : dem Salz im Bereich von 1:1 bis 1:10 ist.

6. Zusammensetzung gemäß einem der Ansprüche 1 bis 5, wobei das Geschmacksmaskierungsmittel aus der Gruppe, bestehend aus Süßungsmitteln, Zuckern und Zuckeralkoholen, ausgewählt ist.

7. Zusammensetzung gemäß einem der Ansprüche 1 bis 5, wobei das Geschmacksmaskierungsmittel aus der Gruppe, bestehend aus Saccharin, Saccharin-Natrium, Aspartam, Cyclamat, Sucralose, Acesulfam, Acesulfam-Kalium, Neotam, Thaumatin, Neohesperidin, Neohesperidin-dihydrochalcon, Saccharose, Trehalose, Lactose, Fruktose, Xylit, Mannit, Sorbit, Isomaltol, L-Arginin, L-Lysin, Citronensäure, Milchsäure, Cineol, Myrtol und Levomenthol, ausgewählt ist.

8. Zusammensetzung gemäß einem der Ansprüche 1 bis 7, umfassend eine weitere Wirkstoffsubstanz, die aus der Gruppe, bestehend aus Chinolonen, Aminoglykosiden, Peptidantibiotika, Monobaktamen, Cefalosporinen, antifungalen Mitteln, Immunmodulatoren, nichtsteroidalen Antirheumatika, Steroiden und pharmazeutisch annehmbaren Salzen davon, ausgewählt ist.

9. Zusammensetzung gemäß einem der Ansprüche 1 bis 8, wobei die Zusammensetzung frei von Cyclodextrinen ist.

10. Verfahren zur Erzeugung eines Aerosols, wobei das Verfahren umfasst:
(a) Bereitstellen einer Zusammensetzung gemäß einem der Ansprüche 1 bis 9;
(b) Bereitstellen eines Aerosol-Generators, der fähig ist, die Zusammensetzung zu aerolisieren, und
(c) Bedienen des Aerosol-Generators, um die Zusammensetzung zu aerolisieren.

11. Verfahren gemäß Anspruch 10, wobei der Aerosol-Generator ein Vernebler des vibrierenden und/oder perforierten vibrierenden Membran-Typs ist.

12. Verfahren gemäß Anspruch 11, wobei der Vernebler zur nasalen oder sinunasalen Verabreichung angepasst wird und in einem kontinuierlichen, durch Atemzug verstärkten, durch Atemzug ausgelösten oder pulsierenden Modus betrieben wird.

13. Verfahren gemäß Anspruch 10, wobei die Zusammensetzung ein polymeres Exzipiens mit bioadhäsiven Eigenschaften enthält, eine dynamische Viskosität von etwa 1 mPa·s bis etwa 10 mPa·s und eine Osmolalität von etwa 200 mOsmol/kg bis etwa 600 mOsmol/kg aufweist, und das Aerosol einen massenmittleren Tröpfchendurchmesser von mehr als etwa 9 µm hat.

14. Verwendung der Zusammensetzung gemäß einem der Ansprüche 1 bis 9 für die Herstellung eines Medikaments für die Prophylaxe oder Behandlung von Krankheiten oder Zuständen des unteren und oberen respiratorischen Trakts oder von Infektionen oder Entzündung der Nase oder der oropharyngealen Regionen.

15. Feste pharmazeutische Zusammensetzung zur Herstellung einer flüssigen Zusammensetzung gemäß einem der Ansprüche 1 bis 9, wobei die feste Zusammensetzung umfasst: (i) ein Makrolid mit schlechtem Geschmack und schlechter chemischer Stabilität in wässriger Lösung; (ii) wenigstens ein Salz, ausgewählt aus der Gruppe, bestehend aus Natriumglukonat, Natriumaspartat, Natriumacetat, Natriumlactat, Natriumsuccinat, Natriummaleat, Magnesiumglukonat, Magnesiumaspartat, Magnesiumcitrat, Magnesiumacetat, Magnesiumlactat, Magnesiumsuccinat und Magnesiummaleat, und (iii) ein von dem Salz verschiedenes Geschmacksmaskierungsmittel.

## Revendications

1. Composition pharmaceutique aqueuse liquide pour administration sous forme d'aérosol pour les voies respiratoires, le nez ou la région oropharyngée comprenant (i) un macrolide ayant mauvais goût et une médiocre stabilité chimique en solution aqueuse ; (ii) au moins un sel choisi dans le groupe consistant en le gluconate de sodium, l'aspartate de sodium, l'acétate de sodium, le lactate de sodium, le succinate de sodium, le maléate de sodium, le gluconate de magnésium, l'aspartate de magnésium, le citrate de magnésium, l'acétate de magnésium, le lactate de magnésium, le succinate de magnésium et le maléate de magnésium ; ou les mélanges de ceux-ci et (iii) un agent masquant le goût différent dudit sel ; dans laquelle
(a) la concentration dudit macrolide dans la composition est dans la plage d'environ 0,25 % en poids à environs en poids ;
(b) le rapport molaire dudit macrolide : ledit sel est dans la plage de 1 : 0,5 à environ 1 : 100 ;
(c) le pH de la composition est dans la plage d'environ 3 à 9 ; et
(d) l'osmolalité de la composition est dans la plage d'environ 150 mOsmol/kg à environ 1 500 mOsmol/kg.

2. Composition selon la revendication 1, ayant une stabilité telle que la concentration du macrolide après stockage pendant trois ans à 4 °C à 8 °C est au moins de 90 % de la concentration initiale.

3. Composition selon la revendication 1 ou 2, dans laquelle le macrolide est l'azithromycine ou la clarithromycine ou un sel pharmaceutiquement acceptable de celles-ci.

4. Composition selon l'une quelconque des revendications 1 à 3, dans laquelle le sel est choisi dans le groupe consistant en le gluconate de sodium, le gluconate de magnésium, le citrate de magnésium, le succinate de sodium, le maléate de sodium, le succinate de magnésium et le maléate de magnésium.

5. Composition selon l'une quelconque des revendications 1 à 4, dans laquelle le rapport molaire dudit macrolide : ledit sel est dans la plage de 1 : 1 à 1 : 10.

6. Composition selon l'une quelconque des revendications 1 à 5, dans laquelle l'agent masquant le goût est choisi dans le groupe consistant en les édulcorants, les sucres et les alcools de sucre.

7. Composition selon l'une quelconque des revendications 1 à 5, dans laquelle l'agent masquant le goût est choisi dans le groupe consistant en la saccharine, la saccharine sodium, l'aspartame, le cyclamate, le sucralose, l'acésulfame, l'acésulfame potassium, le néotame, la thaumatine, la néohespéridine, la néohespéridine dihydrochalcone, le saccharose, le tréhalose, le lactose, le fructose, le xylitol, le mannitol, le sorbitol, l'isomaltol, la L-arginine, la L-lysine, l'acide citrique, l'acide lactique, le cinéol, le myrtol et le lévomenthol.

8. Composition selon l'une quelconque des revendications 1 à 7, comprenant en outre une substance médicamenteuse choisie dans le groupe consistant en les quinolones, les aminoglycosides, les antibiotiques peptidiques, les monobactames, les céfalosporines, les antifongiques, les immunomodulateurs, les anti-inflammatoires non stéroïdiens, les stéroïdes, et les sels pharmaceutiquement acceptables de ceux-ci.

9. Composition selon l'une quelconque des revendications 1 à 8, dans laquelle la composition est dépourvue de cyclodextrines.

10. Procédé de génération d'un aérosol, ledit procédé comprenant :
(a) la fourniture d'une composition selon l'une quelconque des revendications 1 à 9 ;
(b) la fourniture d'un générateur d'aérosol capable de convertir en aérosol la composition ; et
(c) l'actionnement dudit générateur d'aérosol pour convertir en aérosol la composition.

11. Procédé selon la revendication 10, dans lequel le générateur d'aérosol est un nébuliseur de type membrane en vibration et/ou en vibration perforée.

12. Procédé selon la revendication 11, dans lequel le nébuliseur est adapté pour une administration nasale ou sinunasale et actionné en mode continu, amplifié par
la respiration, déclenché par la respiration ou pulsatoire.

13. Procédé selon la revendication 10, dans lequel la composition comprend un excipient polymérique aux propriétés bioadhésives, affiche une viscosité dynamique d'environ 1 mPa·s à environ 10 mPa·s et une osmolalité d'environ 200 mOsmol/kg à environ 600 mOsmol/kg et l'aérosol a un diamètre de gouttelette médian en masse de plus d'environ 9 µm.

14. Utilisation de la composition selon l'une quelconque des revendications 1 à 9, pour la fabrication d'un médicament destiné à la prophylaxie ou au traitement de maladies ou d'affections des voies respiratoires inférieures et supérieures, ou d'infections ou d'une inflammation du nez ou des régions oropharyngées.

15. Composition pharmaceutique solide pour préparer une composition liquide selon l'une quelconque des revendications 1 à 9, laquelle composition solide comprend (i) un macrolide ayant mauvais goût et une médiocre stabilité chimique en solution aqueuse ; (ii) au moins un sel choisi dans le groupe consistant en le gluconate de sodium, l'aspartate de sodium, l'acétate de sodium, le lactate de sodium, le succinate de sodium, le maléate de sodium, le gluconate de magnésium, l'aspartate de magnésium, le citrate de magnésium, l'acétate de magnésium, le lactate de magnésium, le succinate de magnésium et le maléate de magnésium ; et (iii) un agent masquant le goût différent dudit sel.
